# EUROPEAN PATENT SPECIFICATION

(11) **EP 4 368 228 B1**
(45) Date of publication and mention of the grant of the patent: **27.08.2025**
(21) Application number: 23210564.3
(22) Date of filing: 17.07.2014
(51) Int. Cl.: A61M 16/06

(54) **A PATIENT INTERFACE SYSTEM FOR TREATMENT OF RESPIRATORY DISORDERS**
PATIENTENSCHNITTSTELLENSYSTEM ZUR BEHANDLUNG VON ATEMWEGSERKRANKUNGEN
SYSTÈME D'INTERFACE PATIENT POUR LE TRAITEMENT DE TROUBLES RESPIRATOIRES

(30) Priority: 17.07.2013 US 201361847415 P; 14.03.2014 US 201461953240 P; 19.06.2014 NZ 14626417
(43) Date of publication of application: 15.05.2024
(62) Divisional of application: 19213180.3
(73) Proprietor: ResMed Pty Ltd, Bella Vista, NSW 2153 (AU)
(72) Inventor: SMITH, Christopher James, Bella Vista, New South Wales 2153 (AU); KIRBY, Thomas, Bella Vista, New South Wales 2153 (AU); BENTLEY, Alexander, Bella Vista, New South Wales 2153 (AU); BLANCH, Emily Elizabeth, Bella Vista, New South Wales 2153 (AU); HENRY, Robert, Bella Vista, New South Wales 2153 (AU)
(74) Representative: Vossius & Partner Patentanwälte Rechtsanwälte mbB

(56) References cited:
- WO-A1-2009/108995
- WO-A1-2011/022751
- WO-A1-2012/028995

## Description

### FIELD OF THE INVENTION

The present invention relates to a patient interface system to treat a respiratory disorder of a patient.

### BACKGROUND OF THE INVENTION

A range of patient interface systems for treating a respiratory disorder of a patient are disclosed in WO 2011/022751 A1, WO 2009/108992 A1 and WO 2012/028995 A1. WO 20011/022751 A1 discloses a mask system that includes a frame defining a breathing chamber, a cushion provided to the frame and adapted to form a seal with the patient's face, and a shroud provided to the frame and adapted to attach headgear. In some embodiments, the shroud includes upper headgear connectors adapted to attach upper headgear straps, and the frame includes lower headgear connectors adapted to attach lower headgear straps. In other embodiments, the shroud includes upper headgear |connectors adapted to attach upper headgear straps and lower headgear connectors adapted to attach to lower headgear straps.

An aim of the present invention is to improve patient comfort.

### 3.2 DESCRIPTION OF THE RELATED ART

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the air into the venous blood and carbon dioxide to move out. The trachea divides into right and left main bronchi, which further divide eventually into terminal bronchioles. The bronchi make up the conducting airways, and do not take part in gas exchange. Further divisions of the airways lead to the respiratory bronchioles. and eventually to the alveoli. The alveolated region of the lung is where the gas exchange takes place. and is referred to as the respiratory zone. See West, Respiratory Physiology- the essentials.

A range of respiratory disorders exist.

Obstructive Sleep Apnea (OSA). a form of Sleep Disordered Breathing (SDB), is characterized by occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds duration, sometimes 200 to 300 times per night. It often causes excessive daytime somnolence. and it may cause cardiovascular disease and brain damage. The syndrome is a common disorder, particularly in middle aged overweight males, although a person affected may have no awareness of the problem. See US Patent 4,944,310 (Sullivan).

Cheyne-Stokes Respiration (CSR) is a disorder of a patient's respiratory controller in which there are rhythmic altemating periods of waxing and waning ventilation, causing repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent 6,532.959 (Berthon-Jones).

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures. air pollution and genetic factors. Symptoms include: dyspnea on exertion. chronic cough and sputum production.

Neuromuscular Disease (NMD) is a broad term that encompasses many diseases and ailments that impair the functioning of the muscles either directly via intrinsic muscle pathology. or indirectly via nerve pathology. Some NMD patients are characterised by progressive muscular impairment leading to loss of ambulation, being wheelchair-bound. swallowing difficulties, respiratory muscle weakness and, eventually. death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers): (ii) Variable or slowly progressive disorders: Characterised by muscle impairment that worsens over years and.only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness. morning headache. and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion. peripheral oedema, orthopnea, repeated chest infections, morning headaches, fatigue. poor sleep quality and loss of appetite.

Otherwise healthy individuals may take advantage of systems and devices to prevent respiratory disorders from arising.

### 3.2.1 Systems

One known product used for treating sleep disordered breathing is the S9 Sleep Therapy System, manufactured by ResMed.

### 3.2.2 Therapy

Nasal Continuous Positive Airway Pressure (CPAP) therapy has been used to treat Obstructive Sleep Apnea (OSA). The hypothesis is that continuous positive airway pressure acts as a pneumatic splint and may prevent upper airway occlusion by pushing the soft palate and tongue forward and away from the posterior oropharyngeal wall.

Non-invasive ventilation (NIV) has been used to treat OHS, COPD. MD and Chest Wall disorders.

### 3.2.3 Patient Interface

The application of a supply of air at positive pressure to the entrance of the airways of a patient is facilitated by the use of a patient interface. such as a nasal mask, full-face mask or nasal pillows. A range of patient interface devices are known. however a number of them suffer from being one or more of obtrusive, aesthetically undesirable, poorly fitting, difficult to use and uncomfortable especially when worn for long periods of time or when a patient is unfamiliar with a system. Masks designed solely for aviators, as part of personal protection equipment or for the administration of anaesthetics may be tolerable for their original application, but nevertheless be undesirably uncomfortable to be worn for extended periods. for example, while sleeping.

### 3.2.3.1 Seal-forming portion

Patient interfaces typically include a seal-forming portion.

One type of seal-forming portion extends around the periphery of the patient interface, and is intended to seal against the user's face when force is applied to the patient interface with the seal-forming portion in confronting engagement with the user's face. The seal-forming portion may include an air or fluid filled cushion, or a moulded or formed surface of a resilient seal element made of an elastomer such as a rubber. With this type of seal-forming portion, if the fit is not adequate, there will be gaps between the seal-forming portion and the face, and additional force will be required to force the patient interlace against the face in order to achieve a seal.

Another type of scal-forming portion incorporates a flap seal of thin material so positioned about the periphery of the mask so as to provide a self-scaling action against the face of the user when positive pressure is applied within the mask. Like the previous style of seal forming portion, if the match between the face and the mask is not good, additional force may be required to effect a seal, or the mask may leak. Furthermore, if the shape of the seal-forming portion does not match that of the patient. it may crease or buckle in use. giving rise to leaks.

Another form of seal-forming portion may use adhesive to effect a seal. Some patients may find it inconvenient to constantly apply and remove an adhesive to their face.

A range of patient interface seal-forming portion technologies are disclosed in the following patent applications, assigned to ResMed Limited: WO 1998/004.310; WO 2006/074,513: WO 2010/135,785.

### 3.2.3.2 Positioning and stabilising

A seal-forming portion of a patient interface used for positive air pressure therapy is subject to the corresponding force of the air pressure to disrupt a seal. Thus a variety of techniques have been used to position the seal-forming portion, and to maintain it in sealing relation with the appropriate portion of the face.

One technique is the use of adhesives. See for example US Patent publication US 2010/0000534.

Another technique is the use of one or more straps and stabilising harnesses. Many such harnesses suffer from being one or more of ill-fitting, bulky, uncomfortable and awkward to use.

### 3.2.3.3 Vent technologies

Some forms of patient interface systems may include a vent to allow the washout of exhaled carbon dioxide. Many such vents are noisy. Others may block in use and provide insufficient washout. Some vents may be disruptive of the sleep of a bedpartner 1100 of the patient 1000, e.g. through noise or focussed airflow.

ResMed Limited has developed a number of improved mask vent technologies. See WO 1998/034,665; WO 2000/078,381; US 6,581,594; US Patent Application; US 2009/0050156; US Patent Application 2009/0044808.

Table of noise of prior masks (ISO 17510-2:2007. 10 cmH₂O pressure at 1m)

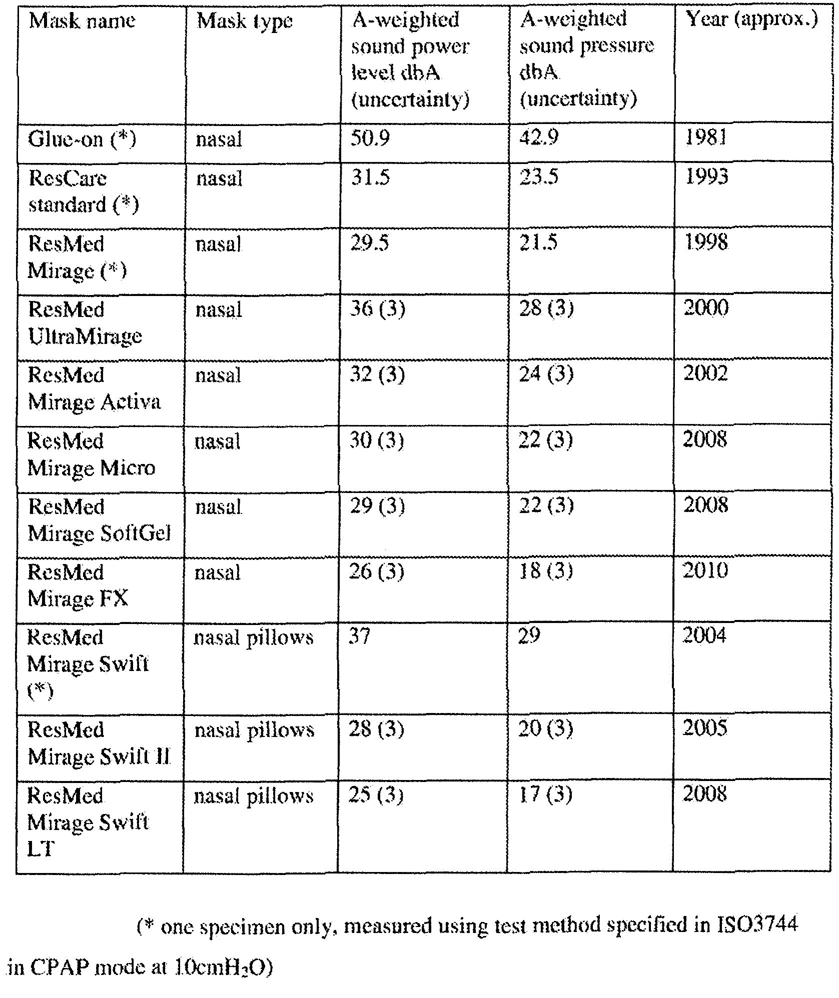

Sound pressure values of a variety of objects are listed below

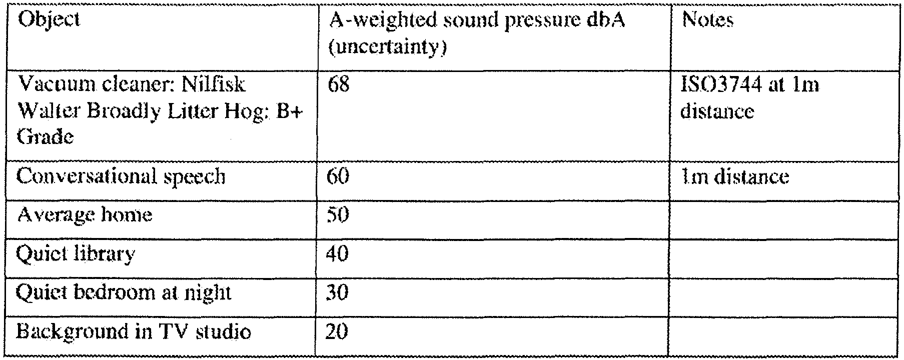

### 3.2.3.4 Nasal pillow technologies

One form of nasal pillow is found in the Adam Circuit manufactured by Puritan Bennett. Another nasal pillow. or nasal puff is the subject of US Patent 4,782,832 (Trimble et al.), assigned to Puritan-Bennett Corporation.

ResMed Limited has manufactured the following products that incorporate nasal pillows: SWIFT nasal pillows mask. SWIFT II nasal pillows mask, SWIFT LT nasal pillows mask, SWIFT FX nasal pillows mask and LIBERTY full-face mask. The following patent applications, assigned to ResMed Limited, describe nasal pillows masks: International Patent Application WO2004/073,778 (describing amongst other things aspects of ResMed SWIFT nasal pillows), US Patent Application 2009/0044808 (describing amongst other things aspects of ResMed SWIFT LT nasal pillows); International Patent Applications WO 2005/063,328 and WO 2006/130,903 (describing amongst other things aspects of ResMed LIBERTY full-face mask); International Patent Application WO 2009/052,560 (describing amongst other things aspects of ResMed SWIFT FX nasal pillows).

### SUMMARY OF THE INVENTION

The present invention provides a patient interface system as defined in claim 1. Further preferred embodiments are laid down in the dependent claims 2-15.

The present technology is directed towards providing medical devices used in the diagnosis. amelioration, treatment, or prevention of respiratory disorders having one or more of improved comfort, cost, efficacy, case of use and manufacturability.

A first aspect of the present technology relates to apparatus used in the diagnosis, amelioration, treatment or prevention of a respiratory disorder.

Another aspect of the present technology is directed to a patient interface system to treat a respiratory disorder of a patient. The patient interface system may comprise: a positioning and stabilising structure including a back portion and a pair of upper straps extending from the back portion: and a patient interface including a patient interface frame and a pair of rigidiser arms each connected to the patient interface frame at a connection point located in a plane substantially parallel to the patient's Frankfort horizontal plane. the pair of rigidiser arms further including a pair of upper attachment points, wherein the pair of upper straps are adapted to be releasably attached to the pair of upper attachment points such that. when donned by the patient, the pair of upper straps are vertically offset relative to the upper attachment points and substantially parallel to the patient's Frankfon horizontal plane, and wherein the patient interface does not include a forehead support.

In examples. (a) the patient interface may further comprise a plenum chamber and a seal-forming structure. (b) the plenum chamber and the seal-forming structure may comprise one piece. (c) the plenum chamber may comprise a more rigid material than the seal-forming structure, (d) the plenum chamber may comprise polycarbonate and the seal-forming structure may comprise silicone, (e) the plenum chamber may comprise at least one plenum chamber retention feature, (f) the mask frame may comprise at least one mask frame retention feature each configured to releasably attach the mask frame to the plenum chamber by a releasable connection with a corresponding one of the at least one plenum chamber retention feature, (g) the plenum chamber may comprise four plenum chamber retention features and the mask frame may comprise four corresponding mask frame retention features, (h) the mask frame and the plenum chamber may be releasably attachable by a hard-to-hard connection, (i) the mask frame may be releasably connected to the plenum chamber about a periphery of the plenum chamber, (j) the positioning and stabilising structure may comprise a pair of lower straps and the patient interface frame may comprise a pair of lower attachment points for releasable attachment to a respective lower strap, and each of the pair of upper straps and each of the pair of lower straps may comprise a loop portion of loop material. (k) a hook portion comprising hook material may be attached at a distal end of each of the pair of upper straps and each of the pair of lower straps. (1) each of the pair of upper straps and each of the pair of lower straps may be adapted to be looped around a respective one of the pair of upper attachment points and the pair of lower attachment points such that each respective strap's hook portion is realasably attachable to that strap's loop portion, (m) each loop portion may be wider than each respective hook portion such that when each hook portion is attached to its corresponding loop portion the loop portion shields the patient's skin from the hook portion, (n) the patient interface may comprise a full-face mask. (o) the positioning and stabilising structure may comprise a crown strap to engage with the parietal bone of the patient's skull, and/or (p) the positioning and stabilising structure may comprise a neck piece to engage with the occipital bone of the patient's skull.

Another aspect of the present technology is directed to a patient interface system to treat a respiratory disorder of a patient. The patient interface system may comprise: a positioning and stabilising structure including a back portion and a pair of upper straps extending from the back portion, and a patient interface including a patient interface frame and a pair of rigidiser arms each connected to the patient interface frame at a connection point located in a plane substantially parallel to the patient's Frankfort horizontal plane, the pair of rigidiser arms further including a pair of upper attachment points, wherein the pair of upper straps are adapted to be releasably attached to the pair of upper attachment points such that, when donned by the patient. sealing force vectors generated by the pair of upper straps are vertically offset relative to the upper attachment points and substantially parallel to the patient's Frankfort horizontal plane, and wherein the patient interface does not include a forehead support.

In examples, (a) the patient interface may further comprise a plenum chamber and a seal-forming structure, (b) the plenum chamber and the seal-forming structure may comprise one piece. (c) the plenum chamber may comprise a more rigid material than the seal-forming structure, (d) the plenum chamber may comprise polycarbonate and the seal-forming structure may comprise silicone, (c) the plenum chamber may comprise at least one plenum chamber retention feature, (f) the mask frame may comprise at least one mask frame retention feature each configured to releasably attach the mask frame to the plenum chamber by a releasable connection with a corresponding one of the at least one plenum chamber retention feature, (g) the plenum chamber may comprise four plenum chamber retention features and the mask frame may comprise four corresponding mask frame retention features, (h) the mask frame and the plenum chamber may be releasably attachable by a hard-to-hard connection, (i) the mask frame may be releasably connected to the plenum chamber about a periphery of the plenum chamber, (j) the positioning and stabilising structure may comprise a pair of lower straps and the patient interface frame may comprise a pair of lower attachment points for releasable attachment to a respective lower strap, and each of the pair of upper straps and each of the pair of lower straps may comprise a loop portion of loop material. (k) a hook portion comprising hook material may be attached at a distal end of each of the pair of upper straps and each of the pair of lower straps, (l) each of the pair of upper straps and each of the pair of lower straps may be adapted to be looped around a respective one of the pair of upper attachment points and the pair of lower attachment points such that each respective strap's hook portion is realasably attachable to that strap's loop portion, (m) each loop portion may be wider than each respective hook portion such that when each hook portion is attached to its corresponding loop portion the loop portion shields the patient's skin from the hook portion, (n) the patient interface may comprise a full-face mask. (o) the positioning and stabilising structure may comprise a crown strap to engage with the parietal bone of the patient's skull, and/or (p) the positioning and stabilising structure may comprise a neck piece to engage with the occipital bone of the patient's skull.

Another aspect of the present technology is directed to a patient interface system to treat a respiratory disorder of a patient. The patient interface system may comprise: a patient interface; a positioning and stabilising structure including a pair of upper straps; and a patient interface frame to connect the patient interface to the positioning and **stabilising** structure, the patient interface frame including a pair of rigidiser arms with upper attachment points to releasably attach the pair of upper straps, wherein each rigidiser arm is connected to the patient interface frame at a connection point such that each rigidiser arm is rotatable about an axis that is substantially perpendicular to the patient's Frankfort horizontal plane. and wherein, when donned by the patient. the pair of upper straps are substantially parallel to the patient's Frankfort horizontal.

In examples, (a) the patient interface may further comprise a plenum chamber and a seal-forming structure, (b) the plenum chamber and the seal-forming structure may comprise one piece, (c) the plenum chamber may comprise a more rigid material than the seal-forming structure, (d) the plenum chamber may comprise polycarbonate and the seal-forming structure may comprise silicone. (e) the plenum chamber may comprise at least one plenum chamber retention feature, (f) the mask frame may comprise at least one mask frame retention feature each configured to releasably attach the mask frame to the plenum chamber by a releasable connection with a corresponding one of the at least one plenum chamber retention feature, (g) the plenum chamber may comprise four plenum chamber retention features and the mask frame may comprise four corresponding mask frame retention features, (h) the mask frame and the plenum chamber may be releasably attachable by a hard-to-hard connection, (i) the mask frame may be releasably connected to the plenum chamber about a periphery of the plenum chamber. (j) the patient interface may comprise a full-face mask, (k) the patient interface may not include a forehead support, (1) a patient interface system to treat a respiratory disorder of a patient may comprise: a patient interface according to any one of the examples above and further comprising a pair of upper attachment points each located on one of the pair of rigidiser arms and a pair of lower attachment points located on the mask frame: and a positioning and stabilising structure including a back portion, a pair of upper straps extending from the back portion, and a pair of lower straps extending from the back portion, (m) each of the pair of upper straps and each of the pair of lower straps may comprise a loop portion of loop material, (n) a hook portion comprising hook material may be attached at a distal end of each of the pair of upper straps and each of the pair of lower straps, (o) each of the pair of upper straps and each of the pair of lower straps may be adapted to be looped around a respective one of the pair of upper attachment points and the pair of lower attachment points such that each respective strap's hook portion is realasably attachable to that strap's loop portion, (p) each loop portion may be wider than each respective hook portion such that when each hook portion is attached to its corresponding loop portion the loop portion shields the patient's skin from the hook portion, (q) the positioning and stabilising structure may comprise a crown strap to engage with the parietal bone of the patient's skull, and/or (r) the positioning and stabilising structure may comprise a neck piece to engage with the occipital bone of the patient's skull.

Another aspect of the present technology is directed to a patient interface to treat a respiratory disorder of a patient. The patient interface may comprise: a patient interface; a patient interface frame to connect the patient interface to a positioning and stabilising structure, the patient interface frame including a pair of frame connection features; and a pair of rigidiser arms, each having a rigidiser arm connection feature, wherein the rigidiser arms are connected to the patient interface frame at connection points by direct engagement between respective frame connection features and rigidiser arm connection features, the connection points being encapsulated by a flexible material.

In examples, (a) the patient interface may further comprise a plenum chamber and a seal-forming structure, (b) the plenum chamber and the seal-forming structure may comprise one piece, (c) the plenum chamber may comprise a more rigid material than the seal-forming structure, (d) the plenum chamber may comprise polycarbonate and the seal-forming structure may comprise silicone. (e) the plenum chamber may comprise at least one plenum chamber retention feature, (f) the mask frame may comprise at least one mask frame retention feature each configured to releasably attach the mask frame to the plenum chamber by a releasable connection with a corresponding one of the at least one plenum chamber retention feature. (g) the plenum chamber may comprise four plenum chamber retention features and the mask frame may comprise four corresponding mask frame retention features. (h) the mask frame and the plenum chamber may be releasably attachable by a hard-to-hard connection, (i) the mask frame may be releasably connected to the plenum chamber about a periphery of the plenum chamber. (j) the patient interface may comprise a full-face mask, (k) the patient interface may not include a forehead support, (1) a patient interface system to treat a respiratory disorder of a patient may comprise: a patient interface according to any one of the examples above and further comprising a pair of upper attachment points each located on one of the pair of rigidiser arms and a pair of lower attachment points located on the mask frame; and a positioning and stabilising structure including a back portion, a pair of upper straps extending from the back portion, and a pair of lower straps extending from the back portion, (m) each of the pair of upper straps and each of the pair of lower straps may comprise a loop portion of loop material. (n) a hook portion comprising hook material may be attached at a distal end of each of the pair of upper straps and each of the pair of lower straps, (o) each of the pair of upper straps and each of the pair of lower straps may be adapted to be looped around a respective one of the pair of upper attachment points and the pair of lower attachment points such that each respective strap's hook portion is realasably attachable to that strap's loop portion, (p) each loop portion may be wider than each respective hook portion such that when each hook portion is attached to its corresponding loop portion the loop portion shields the patient's skin from the hook portion, (q) the positioning and stabilising structure may comprise a crown strap to engage with the parietal bone of the patient's skull, and/or (r) the positioning and stabilising structure may comprise a neck piece to engage with the occipital bone of the patient's skull.

Another aspect of the present technology is directed to a patient interface system to treat a respiratory disorder of a patient. The patient interface may comprise: a patient interface; a positioning and stabilising structure including a back portion and a pair of upper straps extending from the back portion; and a mask frame for retaining the patient interface against the patient's airways. the mask frame including a pair of rigidiser arms including a pair of upper attachment points each located on one of the pair of rigidiser arms for releasable attachment to a respective upper strap, wherein when the patient interface system is donned by the patient the rigidisers arm are shaped and dimensioned to extend from the mask frame along the patient's cheeks and between the patient's eyes and ears such that each upper strap is connectable to a respective upper attachment point above the cars of the patient.

In examples, (a) the patient interface may further comprise a plenum chamber and a seal-forming structure, (b) the plenum chamber and the seal-forming structure may comprise one piece, (c) the plenum chamber may comprise a more rigid material than the seal-forming structure, (d) the plenum chamber may comprise polycarbonate and the seal-forming structure may comprise silicone, (e) the plenum chamber may comprise at least one plenum chamber retention feature, (f) the mask frame may comprise at least one mask frame retention feature each configured to releasably attach the mask frame to the plenum chamber by a releasable connection with a corresponding one of the at least one plenum chamber retention feature, (g) the plenum chamber may comprise four plenum chamber retention features and the mask frame may comprise four corresponding mask frame retention features. (h) the mask frame and the plenum chamber may be releasably attachable by a hard-to-hard connection, (i) the mask frame may be releasably connected to the plenum chamber about a periphery of the plenum chamber. (j) each of the pair of upper straps and each of the pair of lower straps may comprise a loop portion of loop material, (k) a hook portion comprising hook material may be attached at a distal end of each of the pair of upper straps and each of the pair of lower straps, (1) each of the pair of upper straps and each of the pair of lower straps may be adapted to be looped around a respective one of the pair of upper attachment points and the pair of lower attachment points such that each respective strap's hook portion is realasably attachable to that strap's loop portion, (m) each loop portion may be wider than each respective hook portion such that when each hook portion is attached to its corresponding loop portion the loop portion shields the patient's skin from the hook portion, (n) the patient interface may comprise a full-face mask. (o) the patient interface may not include a forehead support, (p) the positioning and stabilising structure may comprise a crown strap to engage with the parietal bone of the patient's skull, and/or (q) the positioning and stabilising structure may comprise a neck piece to engage with the occipital bone of the patient's skull.

Another aspect of the present technology is directed to a patient interface system, comprising: a patient interface having a plenum chamber and a seal-forming structure; a positioning and stabilising structure; and a connection points for attaching the positioning and stabilising structure to the patient interface.

In examples. (a) the plenum chamber and the seal-forming structure may be one-piece. e.g., permanently connected, integrally molded, and/or joined by comolding, (b) the plenum chamber and the seal-forming structure may be removably attached, e.g., by a clipping mechanism, a snap-fit, a press-fit, and/or a friction fit, (c) the patient interface system may further comprise a patient interface frame, wherein the connection points are formed on the patient interface frame, and wherein the patient interface frame is removably attachable to the plenum chamber, (d) the connection points may be formed integrally with or formed in one piece with or molded to the plenum chamber, and/or (e) the positioning and stabilising structure may include upper and lower side straps that join the positioning and stabilising structure to the patient interface at the connection points to releasably secure the patient interface system on the patient's head.

Of course, portions of the examples or aspects may form sub-aspects or sub-cxamples of the present technology. Also, various ones of the examples. sub-aspects and/or aspects may be combined in various manners and also constitute additional examples, sub-examples, aspects or sub-aspects of the present technology.

Other features of the technology will be apparent from consideration of the information contained in the following detailed description. abstract, drawings and claims.

### 5 BRIEF DESCRIPTION OF THE SEVERAL VIEWS OF THE DRAWINGS

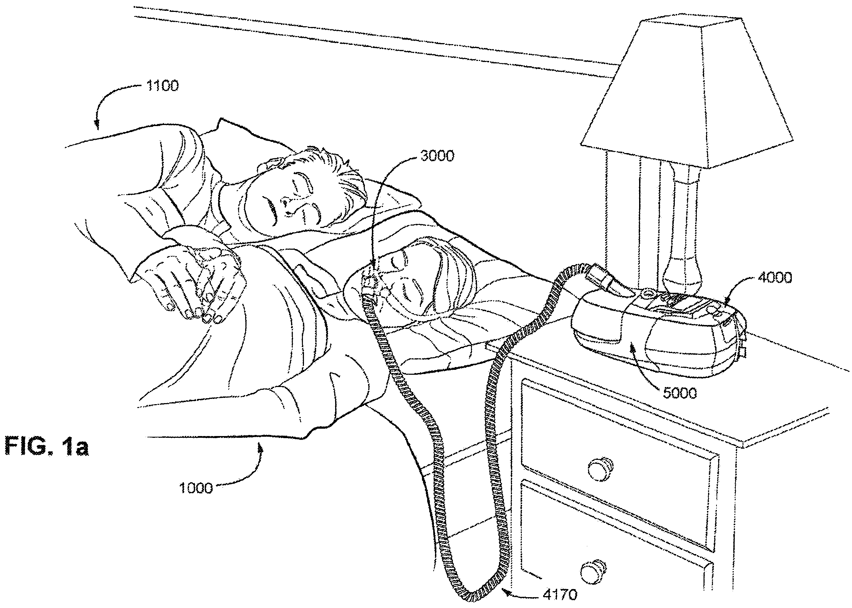

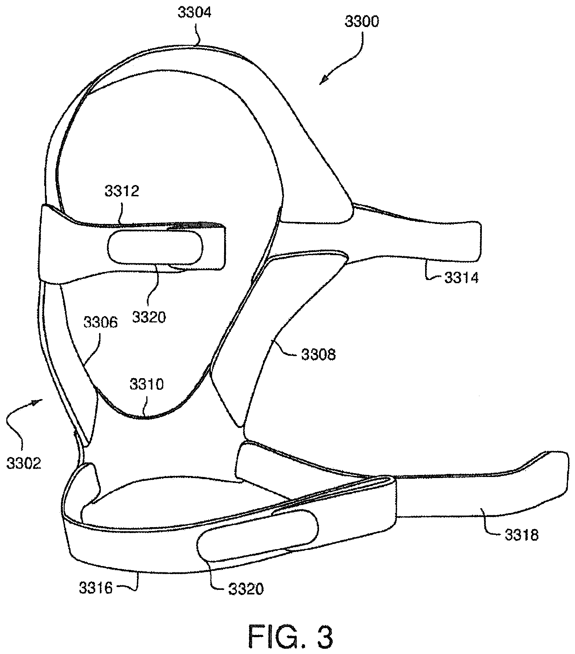

The present technology is illustrated by way of example, and not by way of limitation, in the figures of the accompanying drawings, in which like reference numerals refer to similar elements including:
5.1 TREATMENT SYSTEMS
   Fig. 1a shows a system in accordance with the present technology. A patient 1000 wearing a patient interface 3000, receives a supply of air at positive pressure from a PAP device 4000. Air from the PAP device is humidified in a humidifier 5000, and passes along an air circuit 4170 to the patient 1000.
5.2 THERAPY
**5.2.1 Respiratory system**
   Fig. 2a shows an overview of a human respiratory system including the nasal and oral cavitics, the larynx. vocal folds, oesophagus. trachea. bronchus, long, alveolar sacs, heart and diaphragm.
   Fig. 2b shows a view of a human upper airway including the nasal cavity, nasal bone. lateral nasal cartilage, greater alar cartilage, nostril, lip superior, lip inferior. larynx, hard palate. soft palate, oropharynx, tongue, epiglottis, vocal folds, oesophagus and trachea.
**5.2.2 Facial anatomy**
   Fig. 2c is a front view of a face with several features of surface anatomy identified including the lip superior, upper vermillion, lower vermillion, lip inferior, mouth width. endocanthion, a nasal ala, nasolabial sulcus and cheilion.
   Fig. 2d is a side view of a head with several features of surface anatomy identified including glabella, sellion, pronasale, subnasale. lip superior, lip inferior. supramenton, nasal ridge, otobasion superior and otobasion inferior. Also indicated are the directions superior & inferior. and anterior & posterior.
   Fig. 2e is a further side view of a head. The approximate locations of the Frankfort horizontal and nasolabial angle are indicated.
   Fig. 2f shows a base view of a nose.
   Fig. 2g shows a side view of the superficial features of a nose.
   Fig. 2h shows subcutancal structures of the nose. including lateral cartilage, septum cartilage. greater alar cartilage, lesser alar cartilage and fibrofatty tissue.
   Fig. 2i shows a medial dissection of a nose, approximately several millimeters from a sagittal plane, amongst other things showing the septum cartilage and medial crus of greater alar cartilage.
   Fig. 2j shows a front view of the bones of a skull including the frontal, temporal, nasal and zygomatic bones. Nasal concha are indicated, as are the maxilla, mandible and mental protuberance.
   Fig. 2k shows a lateral view of a skull with the outline of the surface of a head, as well as several muscles. The following bones are shown: frontal, sphenoid, nasal, zygomatic. maxilla, mandible, parietal, temporal and occipital. The mental protuberance is indicated. The following muscles are shown: digastricus, masseter sternocleidomastoid and trapezius.
   Fig. 21 shows an anterolateral view of a nose.
5.3 PATIENT INTERFACE
   Fig. 3 shows a perspective view of a positioning and stabilising structure in accordance with one form of the present technology.

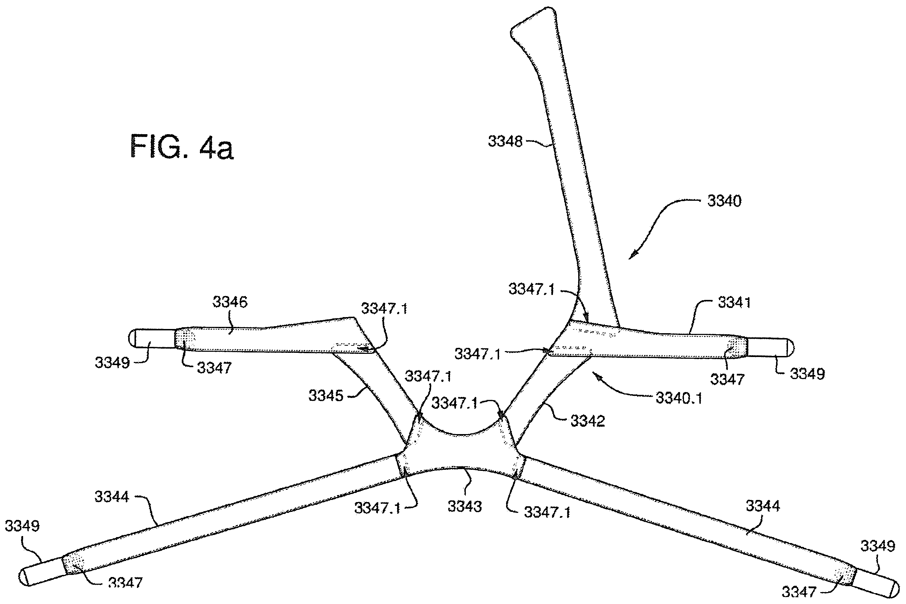

   Fig. 4a shows a rear view of a positioning and stabilising structure assembly laid out flat in accordance with one form of the present technology.

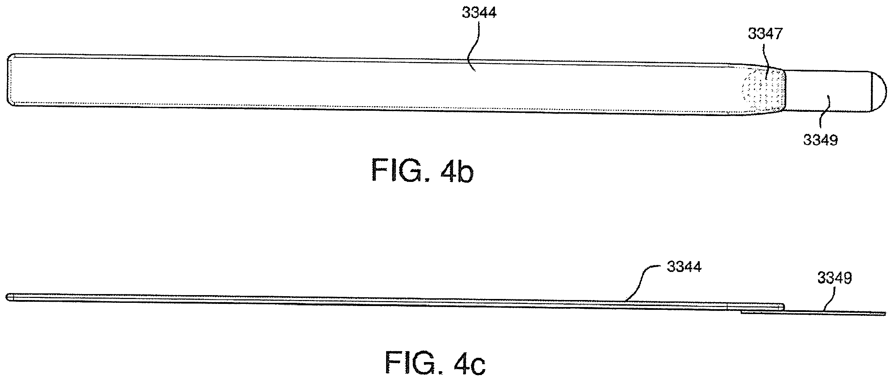

   Fig. 4b shows a rear view of a bottom strap assembly of a positioning and stabilising structure assembly in accordance with one form of the present technology.
   Fig. 4c shows a top view of a bottom strap assembly of a positioning and stabilising structure assembly in accordance with one form of the present technology.

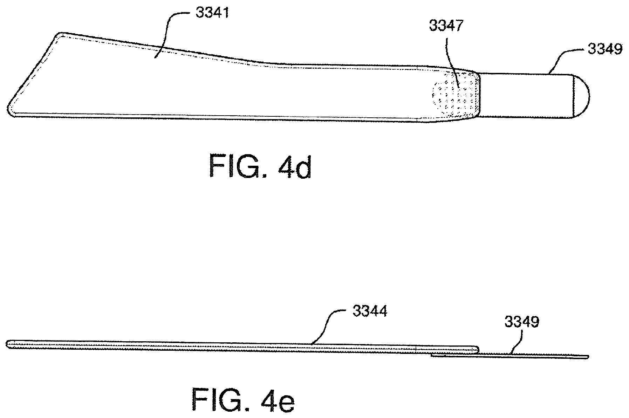

   Fig. 4d shows a rear view of an upper right strap assembly of a positioning and stabilising structure assembly in accordance with one form of the present technology.
   Fig. 4e shows a top view of an upper right strap assembly of a positioning and stabilising structure assembly in accordance with one form of the present technology.

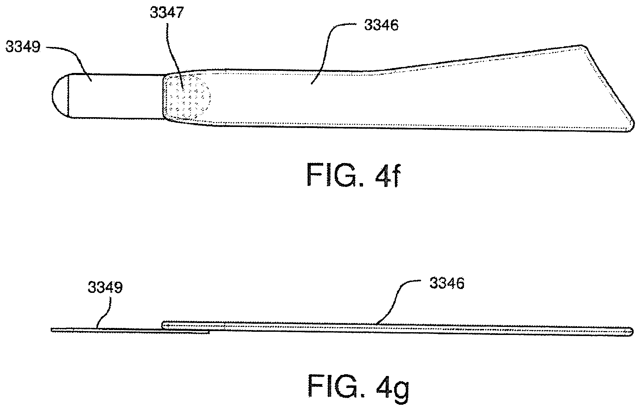

   Fig. 4f shows a rear view of an upper left strap assembly of a positioning and stabilising structure assembly in accordance with one fonn of the present technology.
   Fig. 4g shows a top view of an upper left strap assembly of a positioning and stabilising structure assembly in accordance with one form of the present technology.

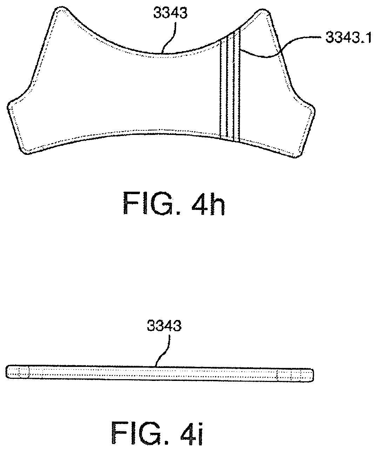

   Fig. 4h shows a rear view of a neck piece of a positioning and stabilising structure assembly in accordance with one form of the present technology.
   Fig. 4i shows a top view of a neck piece of a positioning and stabilising structure assembly in accordance with one form of the present technology.

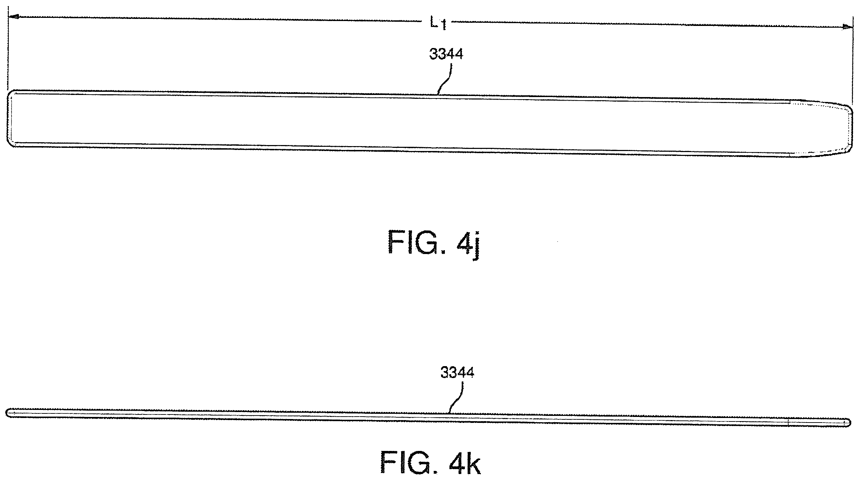

   Fig. 4j shows a rear view of a lower strap of a positioning and stabilising structure assembly in accordance with one form of the present technology.
   Fig. 4k shows a top view of a lower strap of a positioning and stabilising structure assembly in accordance with one form of the present technology.

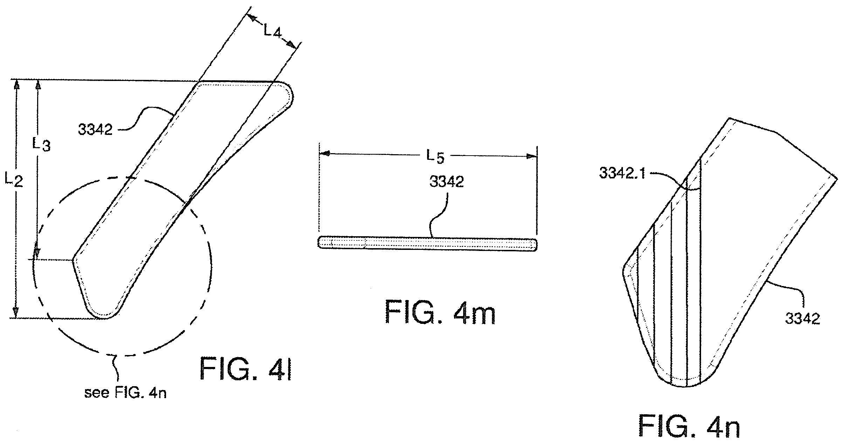

   Fig. 4l shows a rear view of a right crown piece of a positioning and stabilising structure assembly in accordance with one form of the present technology.
   Fig. 4m shows a top view of a right crown piece of a positioning and stabilising structure assembly in accordance with one form of the present technology.
   Fig. 4n shows a detailed rear view of a right crown piece of a positioning and stabilising structure assembly in accordance with one form of the present technology.

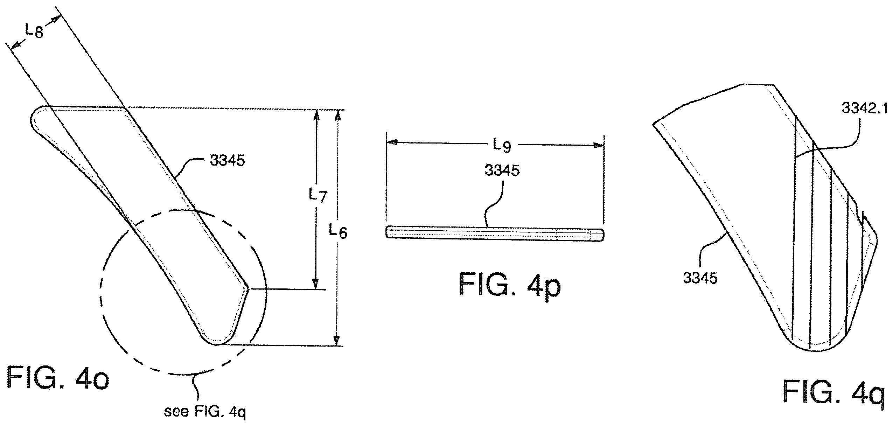

   Fig. 4o shows a rear view of a left crown piece of a positioning and stabilising structure assembly in accordance with one form of the present technology.
   Fig. 4p shows a top view of a left crown piece of a positioning and stabilising structure assembly in accordance with one form of the present technology.
   Fig. 4q shows a detailed rear view of a left crown piece of a positioning and stabilising structure assembly in accordance with one form of the present technology.

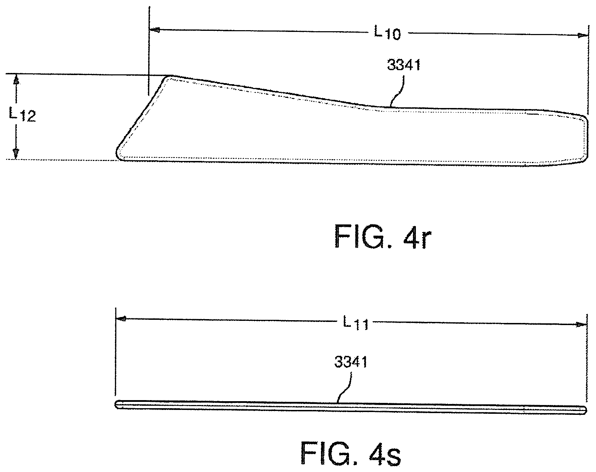

   Fig. 4r shows a rear view of an upper right strap of a positioning and stabilising structure assembly in accordance with one form of the present technology.
   Fig. 4s shows a top view of an upper right strap of a positioning and stabilising structure assembly in accordance with one form of the present technology.
   Fig. 4l shows a rear view of an upper left strap of a positioning and stabilising structure assembly in accordance with one form of the present technology.

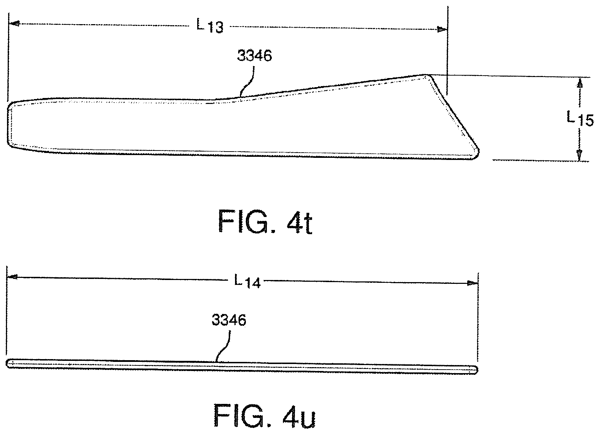

   Fig. 4u shows a top view of an upper left strap of a positioning and stabilising structure assembly in accordance with one form of the present technology.

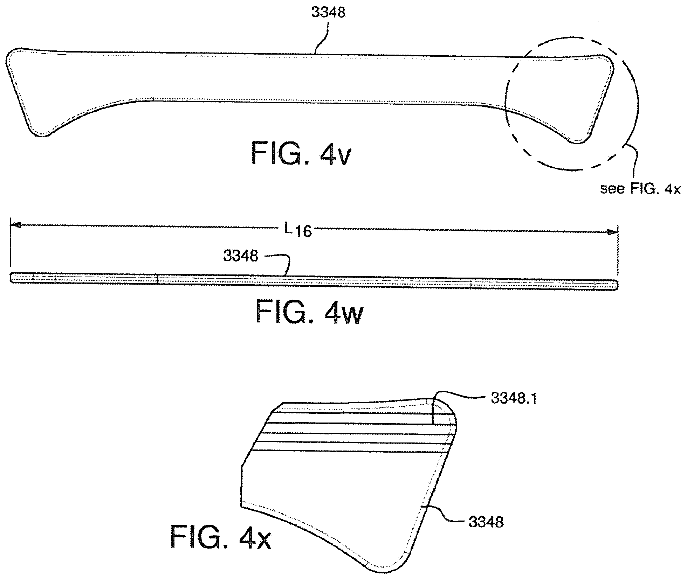

   Fig. 4v shows a rear view of a crown strap of a positioning and stabilising structure assembly in accordance with one form of the present technology.
   Fig. 4w shows a top view of a crown strap of a positioning and stabilising structure assembly in accordance with one form of the present technology.
   Fig. 4x shows a detailed rear view of a crown strap of a positioning and stabilising structure assembly in accordance with one form of the present technology.

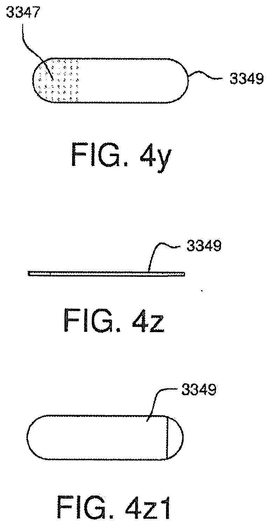

   Fig. 4y shows a rear view of a hook portion of a positioning and stabilising structure assembly in accordance with one form of the present technology.
   Fig. 4z shows a top view of a hook portion of a positioning and stabilising structure assembly in accordance with one form of the present technology.
   Fig. 4z1 shows a rear view of a hook portion of a positioning and stabilising structure assembly in accordance with one form of the present technology.

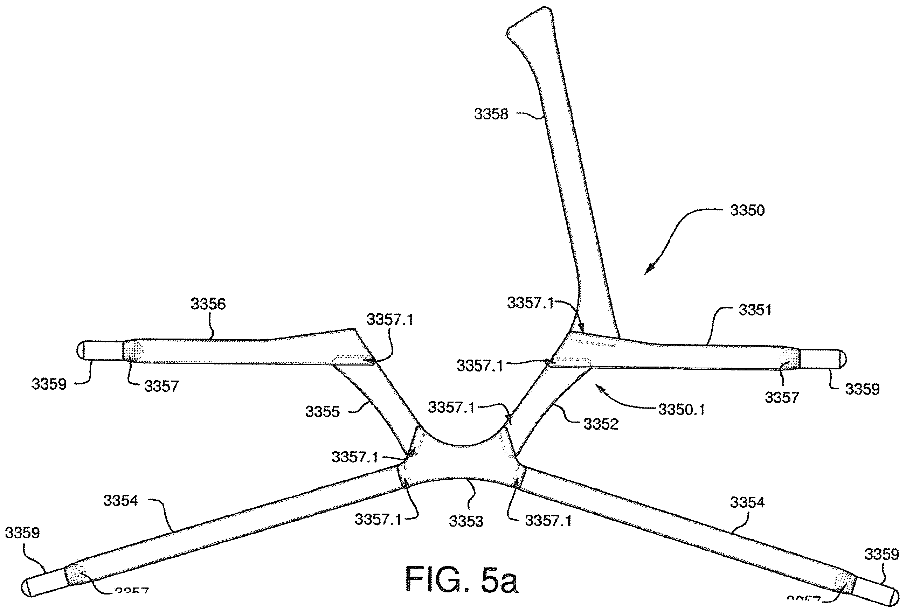

   Fig. 5a shows a rear view of a positioning and stabilising structure assembly laid out flat in accordance with one form of the present technology.

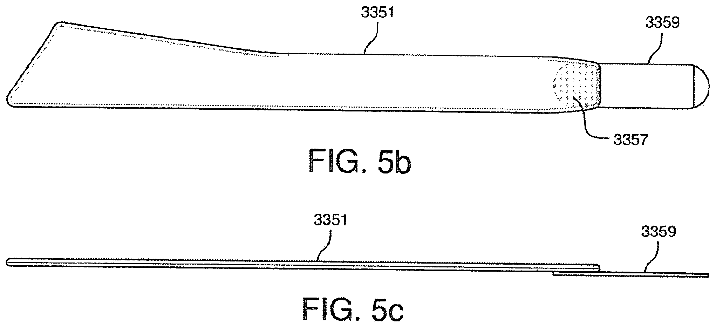

   Fig. 5b shows a rear view of an upper right strap assembly of a positioning and stabilising structure assembly in accordance with one form of the present technology.
   Fig. 5c shows a top view of an upper right strap assembly of a positioning and stabilising structure assembly in accordance with one form of the present technology.

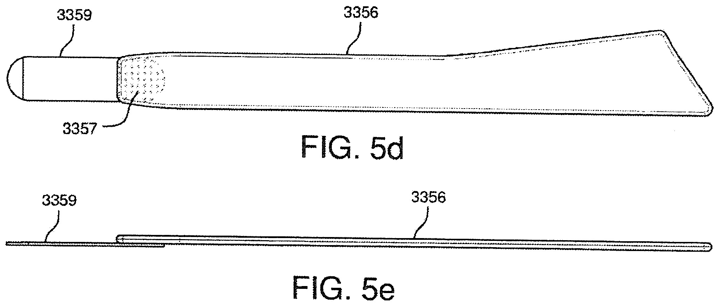

   Fig. 5d shows a rear view of an upper left strap assembly of a positioning and stabilising structure assembly in accordance with one form of the present technology.
   Fig. 5e shows a top view of an upper left strap assembly of a positioning and stabilising structure assembly in accordance with one form of the present technology.

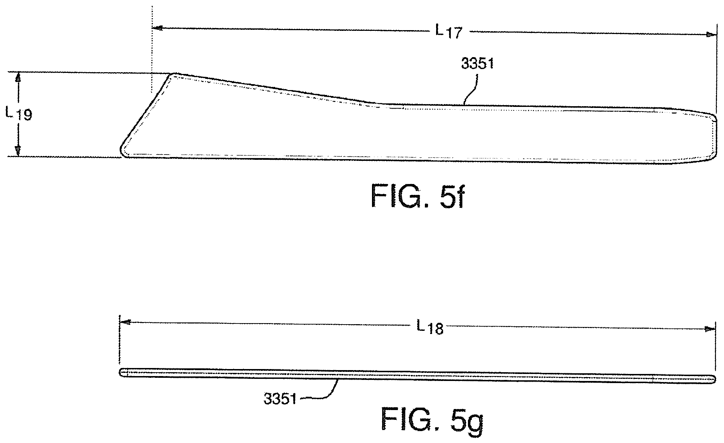

   Fig. 5f shows a rear view of an upper right strap of a positioning and stabilising structure assembly in accordance with one form of the present technology.
   Fig. 5g shows a top view of an upper right strap of a positioning and stabilising structure assembly in accordance with one form of the present technology.

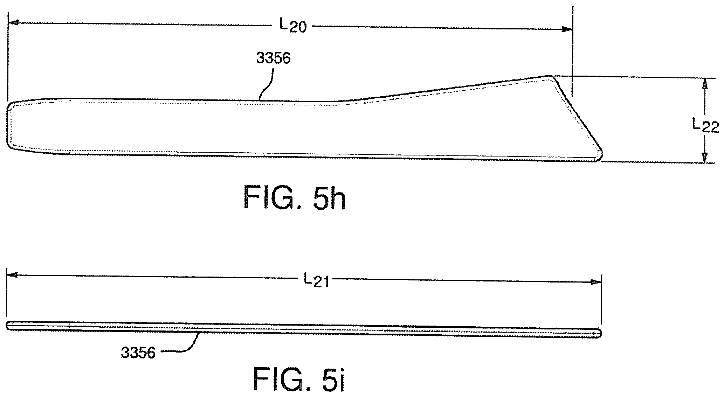

   Fig. 5h shows a rear view of an upper left strap of a positioning and stabilising structure assembly in accordance with one form of the present technology.
   Fig. 5i shows a top view of an upper left strap of a positioning and stabilising structure assembly in accordance with one form of the present technology.

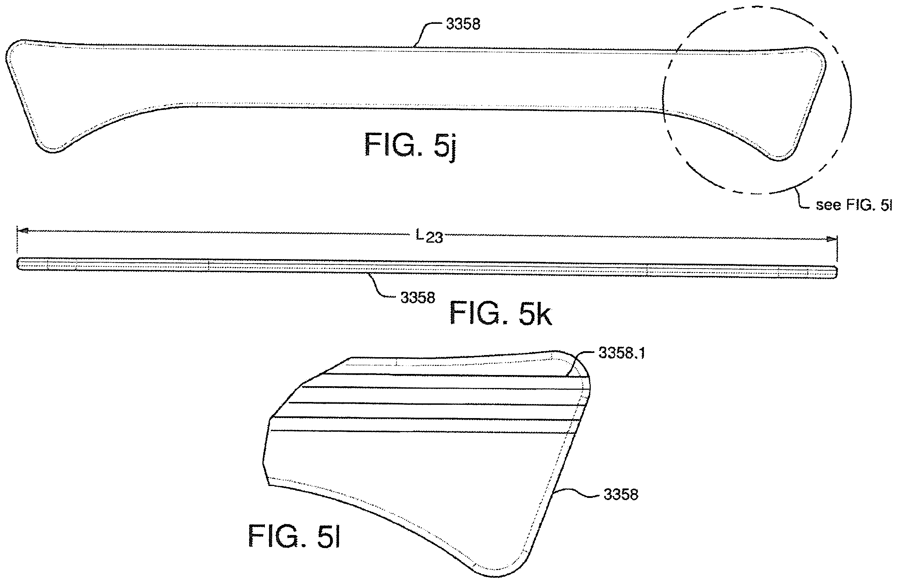

   Fig. 5j shows a rear view of a crown strap of a positioning and stabilising structure assembly in accordance with one form of the present technology.
   Fig. 5k shows a top view of a crown strap of a positioning and stabilising structure assembly in accordance with one form of the present technology.
   Fig. 5l shows a detailed rear view of a crown strap of a positioning and stabilising structure assembly in accordance with one form of the present technology.

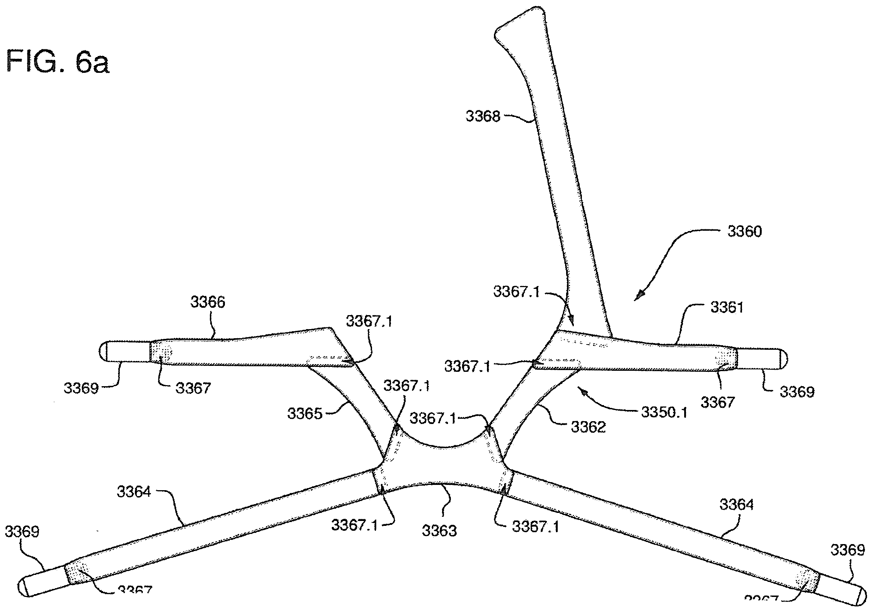

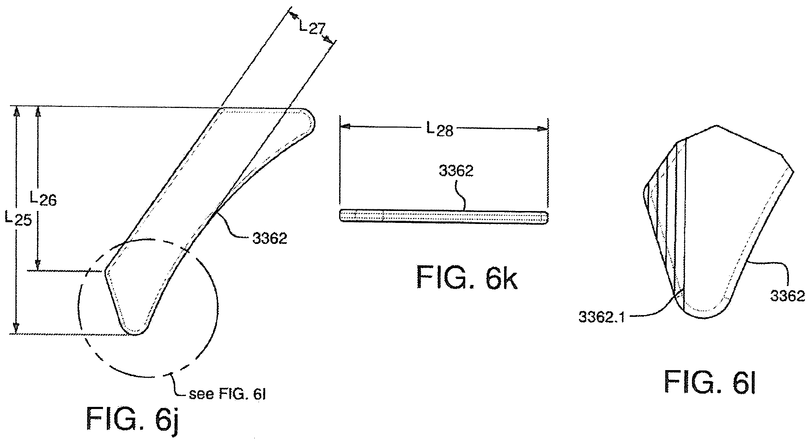

   Fig. 6a shows a rear view of a positioning and stabilising structure assembly laid out flat in accordance with one form of the present technology.

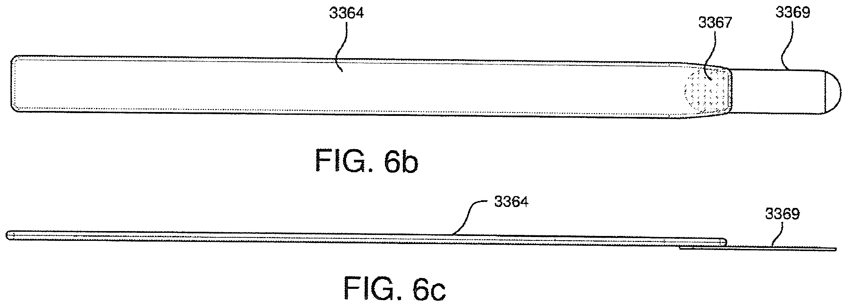

   Fig. 6b shows a rear view of a lower strap assembly of a positioning and stabilising structure assembly in accordance with one form of the present technology.
   Fig. 6c shows a top view of a lower strap assembly of a positioning and stabilising structure assembly in accordance with one form of the present technology.

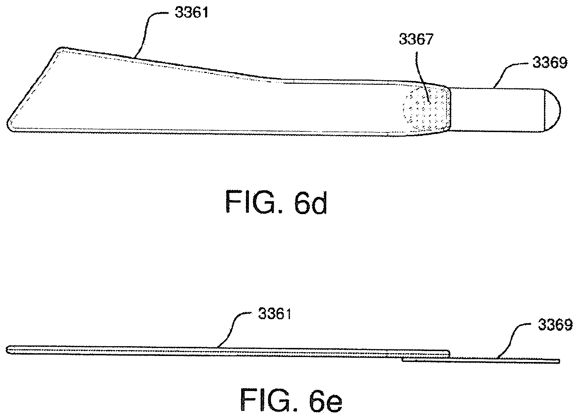

   Fig. 6d shows a rear view of an upper right strap assembly of a positioning and stabilising structure assembly in accordance with one form of the present technology.
   Fig. 6e shows a top view of an upper right strap assembly of a positioning and stabilising structure assembly in accordance with one form of the present technology.

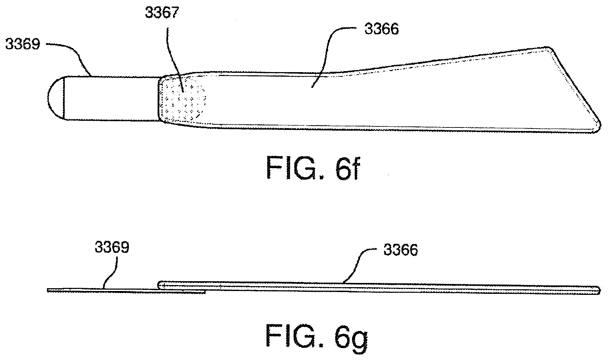

   Fig. 6f shows a rear view of an upper left strap assembly of a positioning and stabilising structure assembly in accordance with one form of the present technology.
   Fig. 6g shows a top view of an upper left strap assembly of a positioning and stabilising structure assembly in accordance with one form of the present technology.

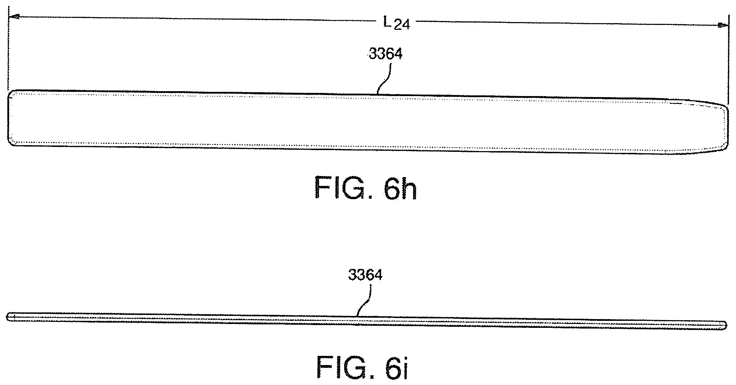

   Fig. 6h shows a rear view of a lower strap of a positioning and stabilising structure assembly in accordance with one form of the present technology.
   Fig. 6i shows a top view of a lower strap of a positioning and stabilising structure assembly in accordance with one form of the present technology.
   Fig. 6j shows a rear view of a right crown piece of a positioning and stabilising structure assembly in accordance with one form of the present technology.
   Fig. 6k shows a top view of a right crown piece of a positioning and stabilising structure assembly in accordance with one form of the present technology.
   Fig. 6I shows a detailed rear view of a right crown piece of a positioning and stabilising structure assembly in accordance with one fonn of the present technology.

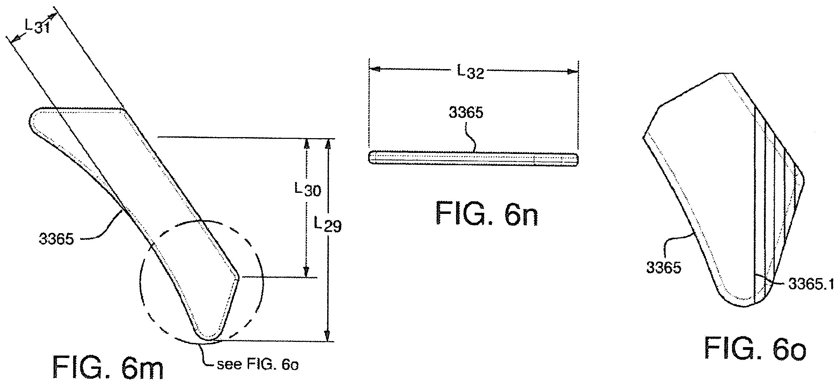

   Fig. 6m shows a rear view of a left crown piece of a positioning and stabilising structure assembly in accordance with one form of the present technology.
   Fig. 6n shows a top view of a left crown piece of a positioning and stabilising structure assembly in accordance with one form of the present technology.
   Fig. 6o shows a detailed rear view of a left crown piece of a positioning and stabilising structure assembly in accordance with one form of the present technology.

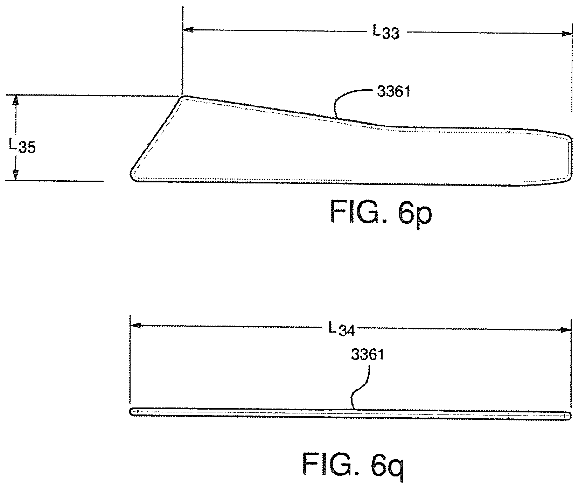

   Fig. 6p shows a rear view of an upper right strap of a positioning and stabilising structure assembly in accordance with one form of the present technology.
   Fig. 6q shows a top view of an upper right strap of a positioning and stabilising structure assembly in accordance with one form of the present technology.

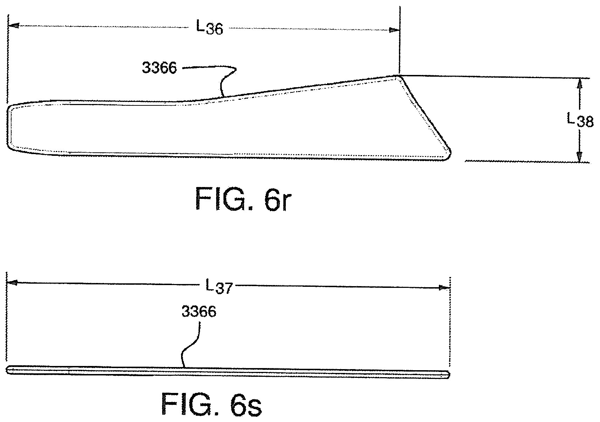

   Fig. 6r shows a rear view of an upper left strap of a positioning and stabilising structure assembly in accordance with one form of the present technology.
   Fig. 6s shows a top view of an upper left strap of a positioning and stabilising structure assembly in accordance with one form of the present technology.

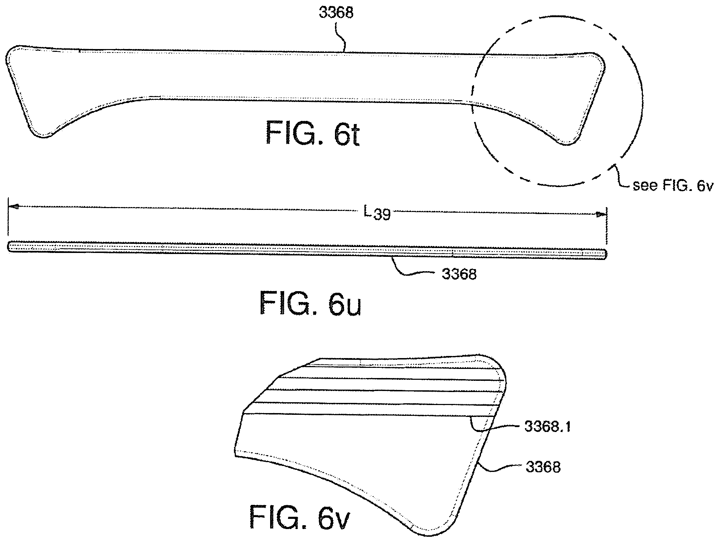

   Fig. 6t shows a rear view of a crown strap of a positioning and stabilising structure assembly in accordance with one form of the present technology.
   Fig. 6u shows a top view of a crown strap of a positioning and stabilising structure assembly in accordance with one form of the present technology.
   Fig. 6v shows a detailed rear view of a crown strap of a positioning and stabilising structure assembly in accordance with one form of the present technology.

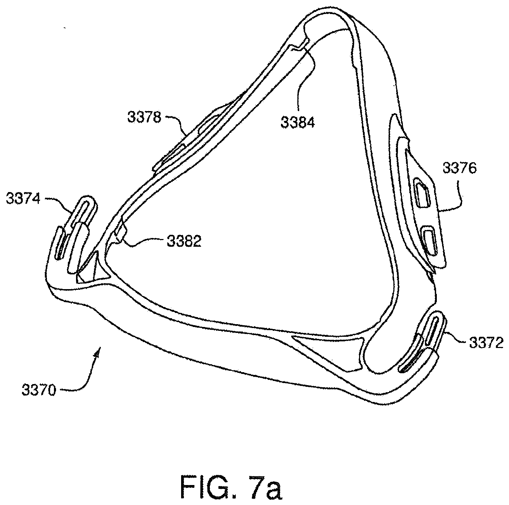

   Fig. 7a shows a bottom perspective view of a patient interface frame in accordance with one form of the present technology.

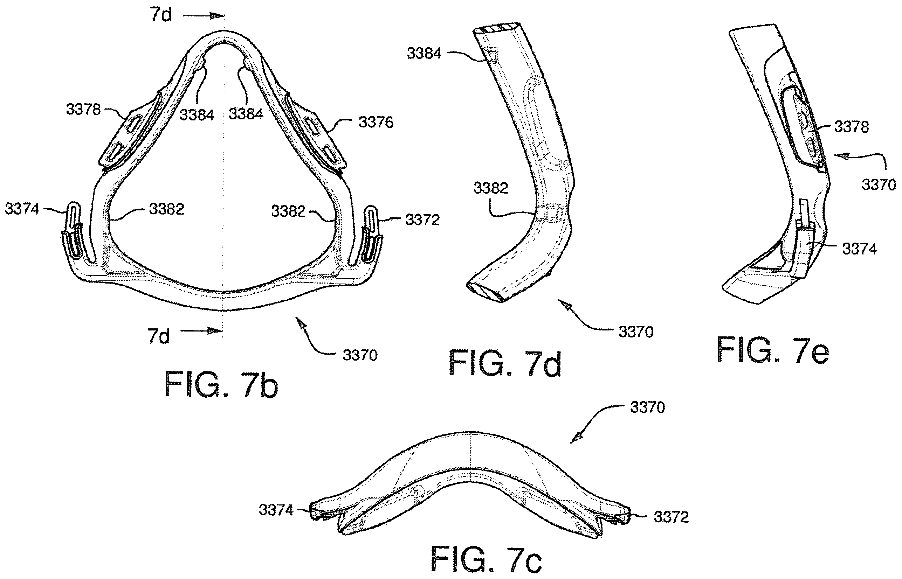

   Fig. 7b shows a front view of a patient interface frame in accordance with one form of the present technology.
   Fig. 7c shows a bottom view of a patient interface frame in accordance with one form of the present technology.
   Fig. 7d shows an inner cross-sectional view of a patient interface frame taken through line 7d-7d of Fig. 7b in accordance with one form of the present technology.
   Fig. 7e shows an outer cross-sectional view of a patient interface frame taken through line 7d-7d of Fig. 7b in accordance with one form of the present technology.

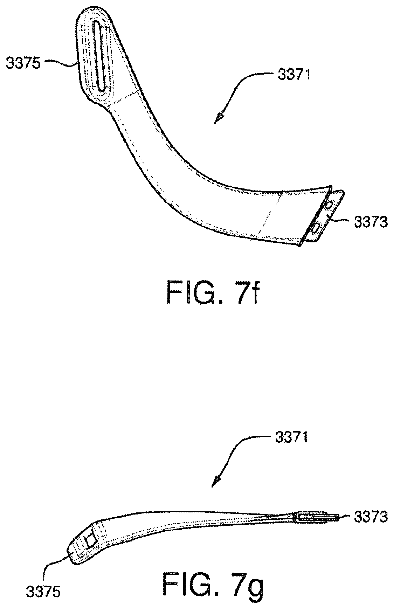

   Fig. 7f shows a front view of a left rigidiser arm in accordance with one form of the present technology.
   Fig. 7g shows a bottom view of a left rigidiser arm in accordance with one form of the present technology.

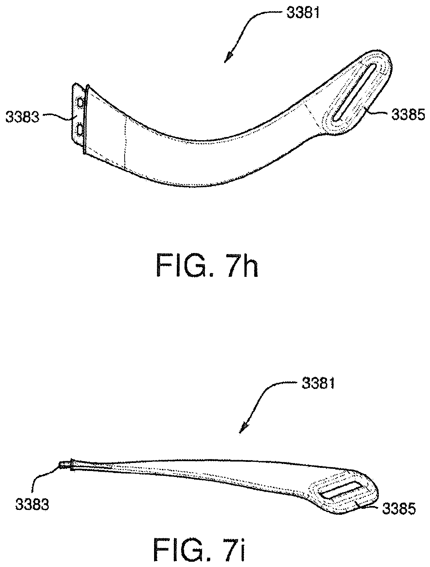

   Fig. 7h shows a front view of a right rigidiser arm in accordance with one form of the present technology.
   Fig. 7i shows a bottom view of a right rigidiser arm in accordance with one form of the present technology.

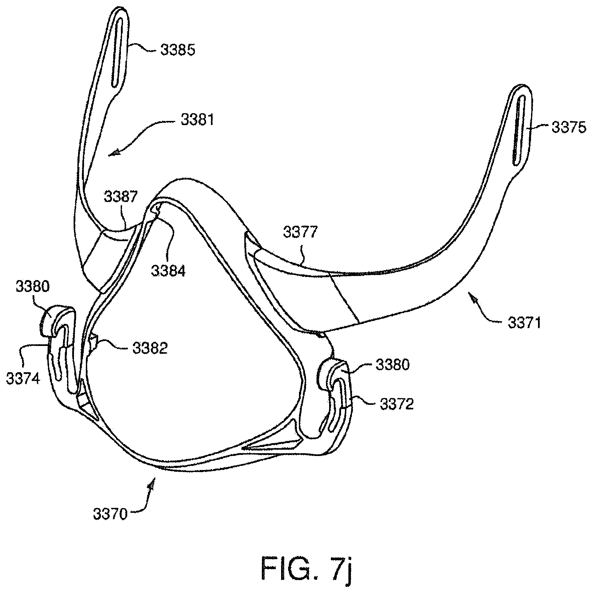

   Fig. 7j shows a perspective view of a patient interface frame assembly in accordance with one form of the present technology.

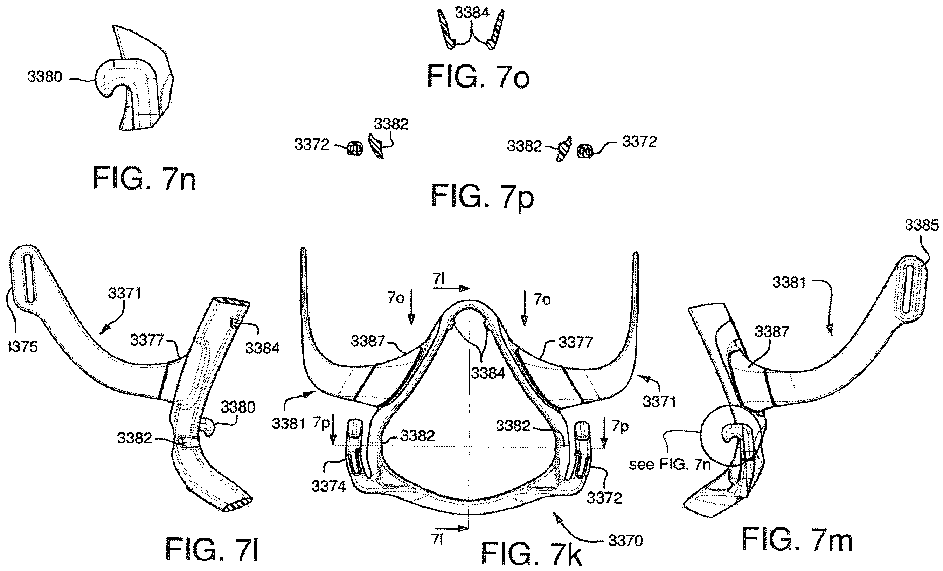

   Fig. 7k shows a front view of a patient interface frame with rigidiser arms in accordance with one form of the present technology.
   Fig. 7l shows an inner cross-sectional view of a patient interface frame taken through line 71-71 of Fig. 7k in accordance with one form of the present technology.
   Fig. 7m shows an outer cross-sectional view of a patient interface frame taken through line 7l-7l of Fig. 7k in accordance with one form of the present technology.
   Fig. 7n shows a detailed view of a hook of an attachment point of a patient interface frame in accordance with one form of the present technology.
   Fig. 7o shows a cross-sectional view of a patient interface frame taken through line 7o-7o of Fig. 7k in accordance with one form of the present technology.
   Fig. 7p shows a cross-sectional view of a patient interface frame taken through line 7p-7p of Fig. 7k in accordance with one form of the present technology.

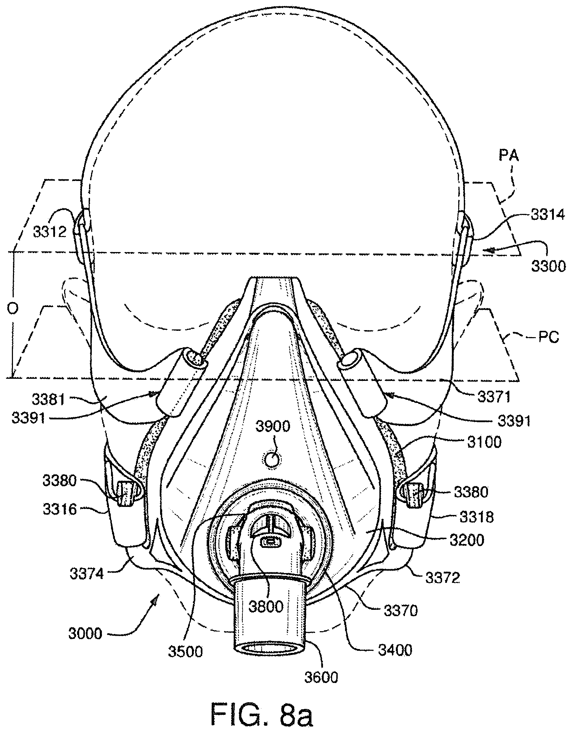

   Fig. 8a shows a front view of a patient interface and a positioning and stabilising structure donned on a patient in accordance with one form of the present technology.

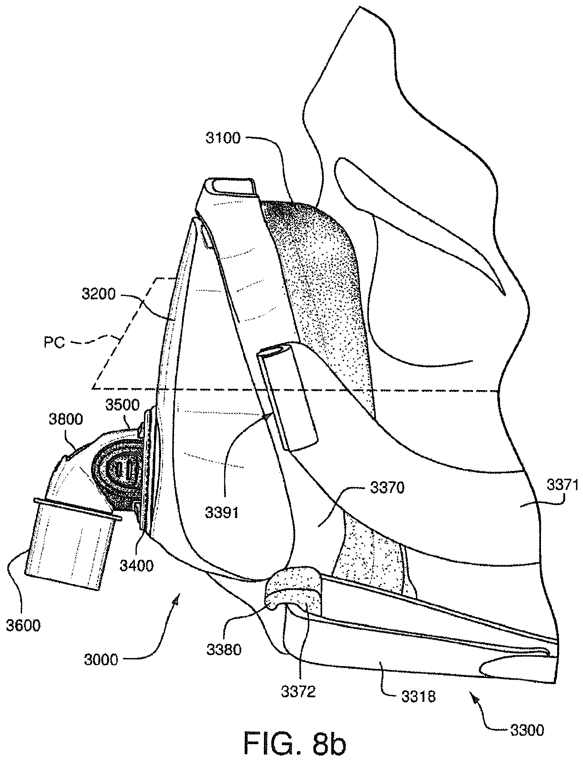

   Fig. 8b shows a detailed side view of a patient interface and a positioning and stabilising structure donned on a patient in accordance with one form of the present technology.

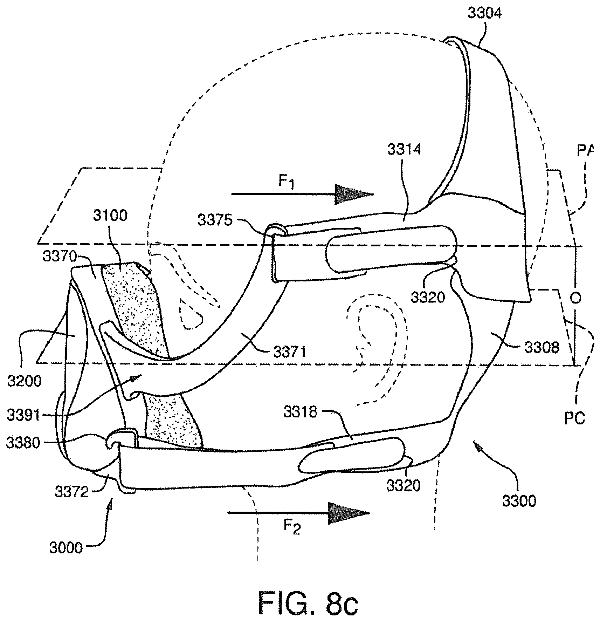

   Fig. 8c shows a side view of a patient interface and a positioning and stabilising structure donned on a patient in accordance with one form of the present technology.

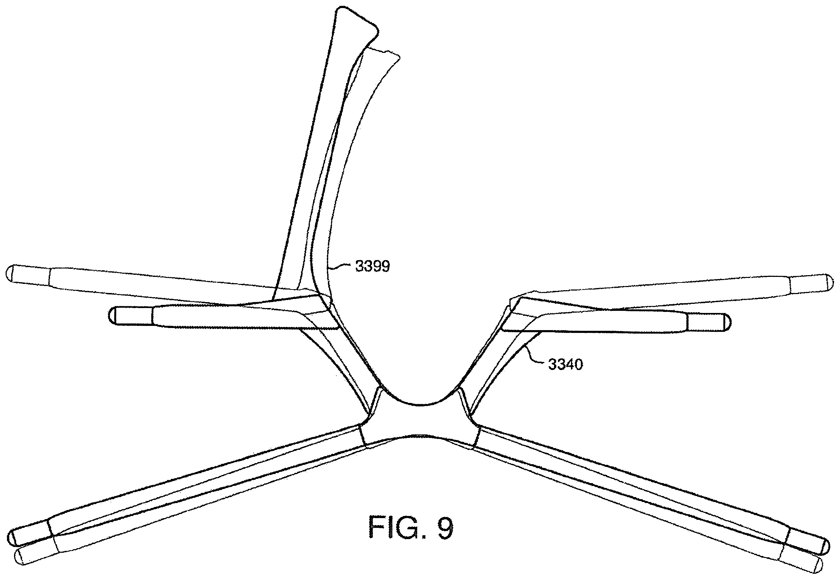

   Fig. 9 shows a comparison of a positioning and stabilising structure laid out flat according to the present technology with a related art positioning and stabilising structure also laid out flat.

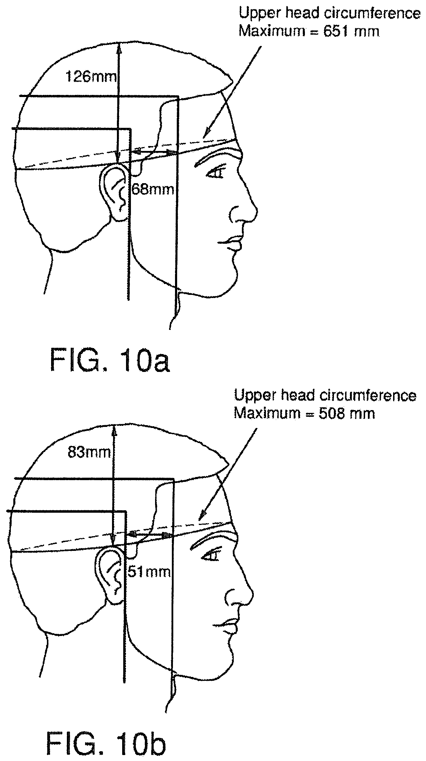

   Fig. 10a shows a side view of measurements of a patient's head and face.
   Fig. 10b shows a side view of measurements of a patient's head and face.

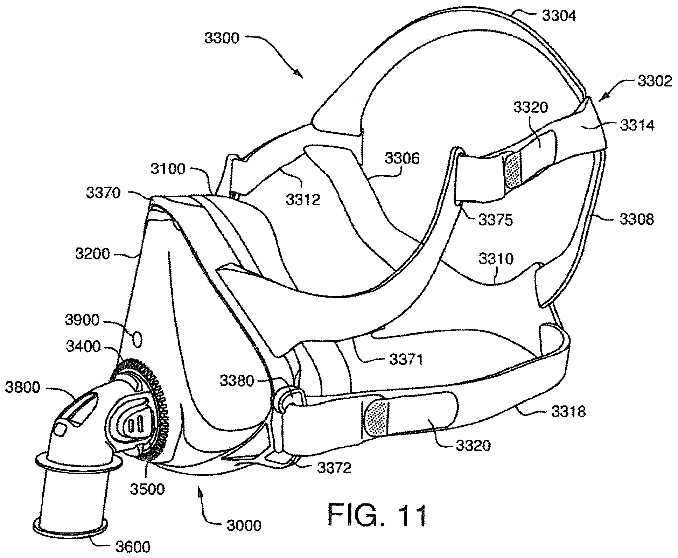

   Fig. 11 shows a perspective view of a patient interface and positioning and stabilising structure according to one form of the present technology.

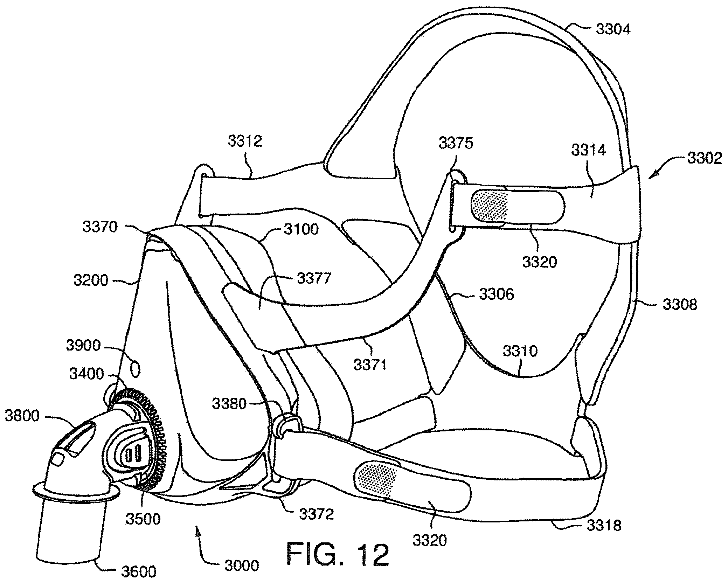

   Fig. 12 shows a perspective view of a patient interface and positioning and stabilising structure according to one form of the present technology.

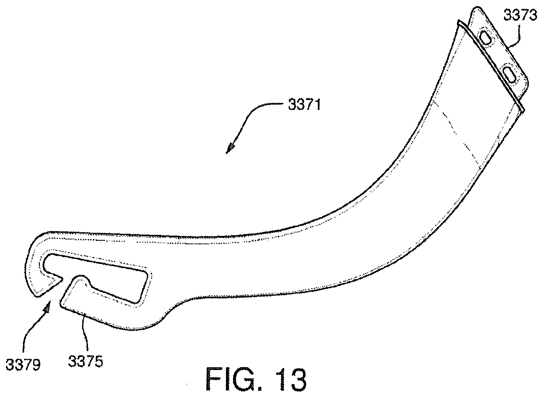

   Fig. 13 shows a perspective view of a rigidiser arm according to one form of the present technology.

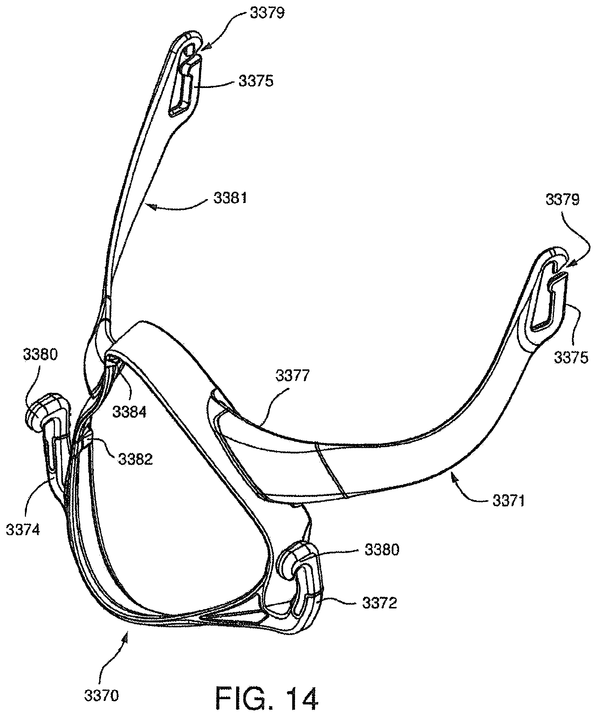

   Fig. 14 shows a perspective view of a patient interface frame and a pair of rigidiser anns according to an example of the present technology.

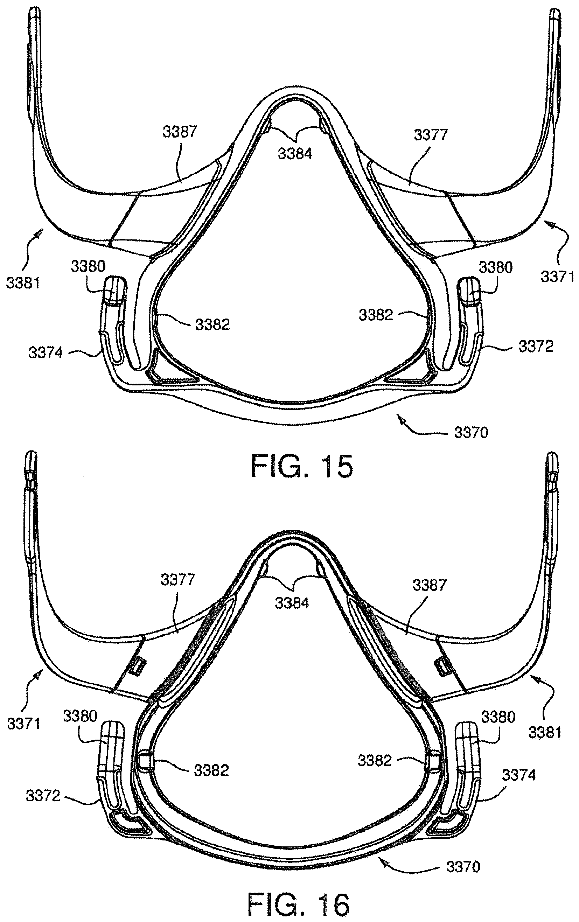

   Fig. 15 shows a front view of a patient interface frame and a pair of rigidiser arms according to an example of the present technology.
   Fig. 16 shows a rear view of a patient interface frame and a pair of rigidiser arms according to an example of the present technology.

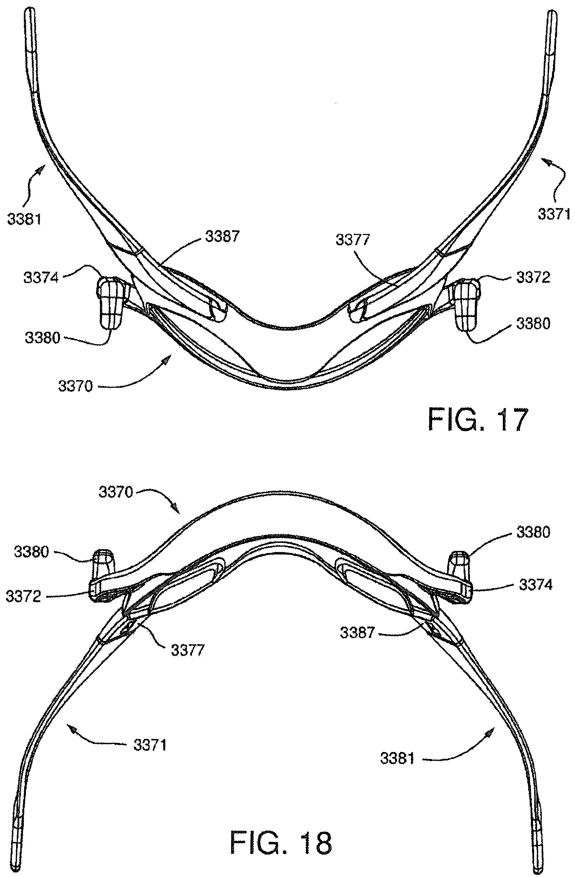

   Fig. 17 shows a top view of a patient interface frame and a pair of rigidiser arms according to an example of the present technology.
   Fig. 18 shows a bottom view of a patient interface frame and a pair of rigidiser arms according to an example of the present technology.

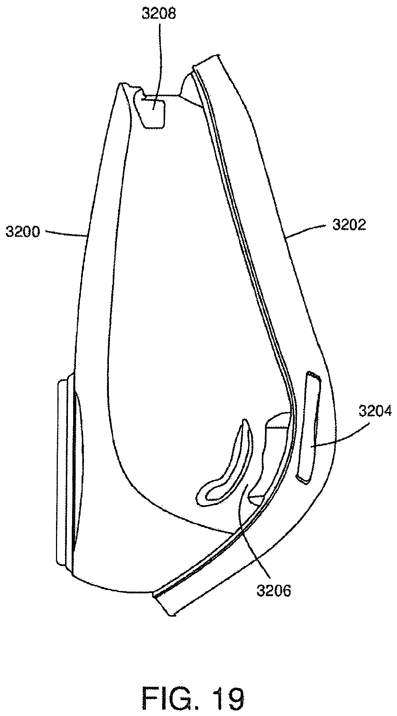

   Fig. 19 shows a side view of a plenum chamber of a patient interface according to an example of the present technology.

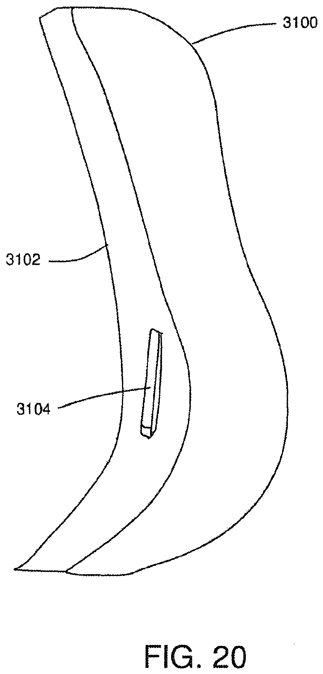

   Fig. 20 shows side view of a seal-forming structure of a patient interface according to an example of the present technology.

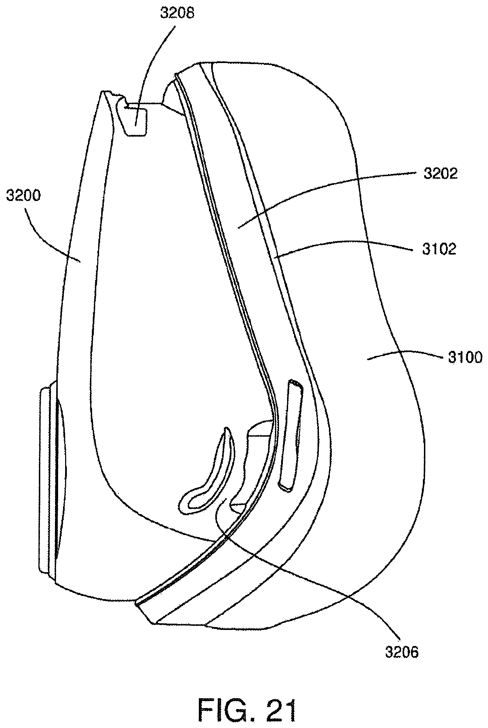

   Fig. 21 shows a side of a patient interface according to an example of the present technology.

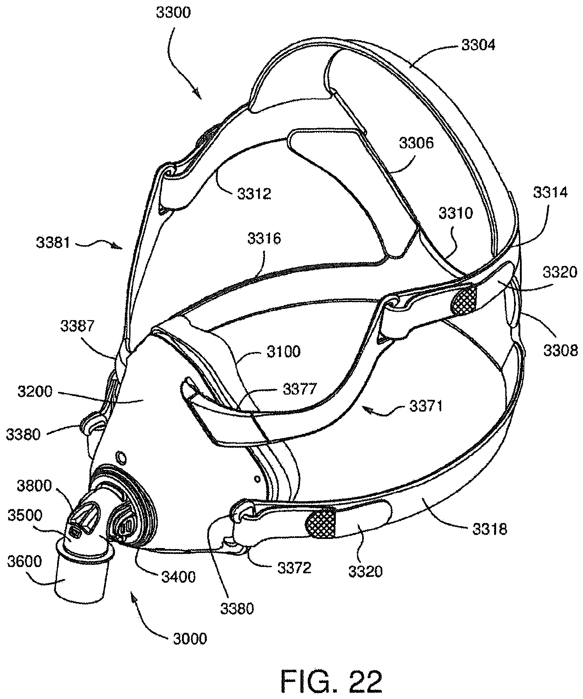

   Fig. 22 shows a perspective view of a patient interface and a positioning and stabilising structure according to an example of the present technology.

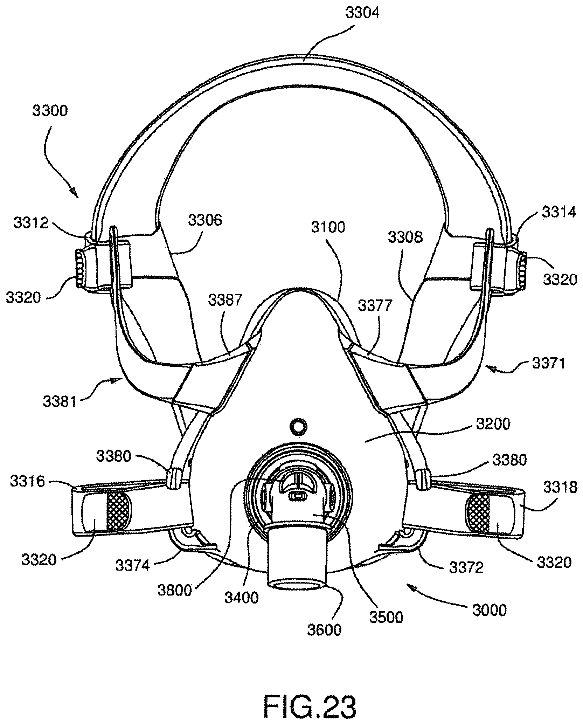

   Fig. 23 shows a front view of a patient interface and a positioning and stabilising structure according to an example of the present technology.

### 6 DETAILED DESCRIPTION OF EXAMPLES OF THE TECHNOLOGY

Before the present technology is described in further detail. it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 6.1 TREATMENT SYSTEMS

In one form, the present technology comprises apparatus for treating a respiratory disorder. The apparatus may comprise a flow generator or blower for supplying pressurised respiratory gas, such as air, to the patient 1000 via an air delivery tube leading to a patient interface 3000.

### 6.2 THERAPY

In one form. the present technology comprises a method for treating a respiratory disorder comprising the step of applying positive pressure to the entrance of the airways of a patient 1000.

### 6.2.1 Nasal CPAP for OSA

In one form, the present technology comprises a method of treating Obstructive Sleep Apnea in a patient by applying nasal continuous positive airway pressure to the patient.

In certain examples of the present technology, a supply of air at positive pressure is provided to the nasal passages of the patient via one or both nares.

In certain examples of the present technology, mouth breathing is limited, restricted or prevented.

### 6.3 PATIENT INTERFACE

A non-invasive patient interface 3000 in accordance with one aspect of the present technology comprises the following functional aspects: a seal-forming structure 3100. a plenum chamber 3200, a positioning and stabilising structure 3300 and a connection port 3600 for connection to air circuit 4170. In some forms a functional aspect may be provided by one or more physical components. In some forms, one physical component may provide one or more functional aspects. In use the seal-forming structure 3100 is arranged to surround an entrance to the airways of the patient so as to facilitate the supply of air at positive pressure to the airways.

The term "patient interface", for the purposes of the present disclosure. is intended to refer to various interface types such as a full face mask, a nasal mask, an oro-nasal mask, nasal puffs or pillows, and/or an oro-nasal mask. In other words. any device that facilitates an interface with an entrance to the patient's airways for the supply of pressurized breathable gas may be a "patient interface". It should also be understood that those skilled in the art would also understood the term "mask" to refer broadly to the various forms of patient interface described above. Thus, for example, a mask may include a full face mask, a nasal mask, an oro-nasal mask, nasal puffs or pillows, and/or an oro-nasal mask.

### 6.3.1 A Full-Face Mask Without A Forehead Support

According to an example of the present technology. a patient interface 3000 may be formed without a forehead support. An exemplary patient interface 3000 may also comprise a full-face mask. By excluding a forehead support from the patient interface 3000 the patient may be provided with a less obstructed view due to the absence of a structure extending upward toward the forehead and between the eyes. This may also make the patient interface 3000 more visually appealing for a bed partner because there is less structure obstructing the face. Additionally, the absence of a forehead support reduces the points of contact on the patient's face that are required by the patient interface 3000. However, the patient interface 3000 must still be able to be urged against the patient's face with a force that is sufficient in magnitude and distribution at the seal-forming structure 3100 so as to ensure an effective pneumatic seal with the patient's airways.

Also, a positioning and stabilising structure 3300 (described in greater detail below) must be designed for use in conjunction with the patient interface 3000 to prevent riding up of the patient interface. Ride up may be described as the upward movement of the patient interface 3000 such that the patient interface comes to rest at a higher position on the patient's face than its initial position. If sealing force vectors generated by upper straps of the positioning and stabilising structure 3300 are generated in a vertical direction, then ride up may occur. Other factors, which will be described in greater detail below, may also contribute to ride up.

### 6.3.1.1 Seal-Forming Structure and Plenum Chamber

According to an example of the present technology, the patient interface 3000 may include the seal-forming structure 3100 and the plenum chamber 3200. Examples of the patient interface 3000 and associated features are depicted in Figs. 8a-8c.

In one form of the present technology. a seal-forming structure 3100 provides a scaling-forming surface, and may additionally provide a cushioning function.

A seal-forming structure 3100 in accordance with the present technology may be constructed from a soft, flexible, resilient material such as silicone.

In one form, the seal-forming structure 3100 comprises a scaling flange and a support flange. The sealing flange comprises a relatively thin member with a thickness of less than about 1mm. for example about 0.25mm to about 0.45mm, that extends around the perimeter of the plenum chamber 3200. The support flange may be relatively thicker than the sealing flange. The support flange may be disposed between the sealing flange and the marginal edge of the plenum chamber 3200, and extends at least part of the way around the perimeter. The support flange may be or may include a spring-like element and functions to support the sealing flange from buckling in use. In use the sealing flange can readily respond to system pressure in the plenum chamber 3200 acting on its underside to urge it into tight sealing engagement with the face.

The plenum chamber 3200 may have a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend, in use, about the entire perimeter of the plenum chamber 3200.

According to an example of the present technology. the seal-forming structure 3100 may be molded onto the plenum chamber 3200 to form one piece. The seal-forming structure 3100 and the plenum chamber 3200 may comprise a patient interface or a mask assembly according to an example of the present technology.

### 6.3.1.2 Positioning and Stabilising Structure

Figs. 3, 4a-4z1, 5a-5l, 6a-6v, 8a-8c. and 9 depict various examples of the positioning and stabilising structure 3300 according to the present technology.

Fig. 3 shows a perspective view of the positioning and stabilising structure 3300 according to an example of the technology. This view shows the positioning and stabilising and structure 3300 as it may be worn by a patient, although no patient interface 3000 is shown.

The exemplary positioning and stabilising structure 3300 shown in Fig. 3 includes a back portion 3302. The exemplary back portion 3302 may include a left crown piece 3306 and a right crown piece 3308, as well as a neck piece 3310. The positioning and stabilising structure 3300 may also include a crown strap 3304. Also. extending from the back portion 3302 may be an upper left strap 3312 and an upper right strap 3314, as well as a lower left strap 3316 and a lower right strap 3318. A hook portion 3320 of hook material may be attached at a distal end of each of the upper straps 3312, 3314 and lower straps 3316, 3318. The hook portion 3320 may be used to attach each strap to itself by looping through attachment points of the patient interface. as will be discussed in greater detail below. The connection with the hook portions 3320 may be facilitated by a layer of loop material on the exterior of some or all of the components of the positioning and stabilising structure 3300. According to one example of the present technology, the upper straps 3312, 3314 and lower straps 3316, 3318 may each have an exterior layer of hook material for releasable attachment with the respective hook portions 3320.

According to another example of the present technology, each component of the positioning and stabilising structure 3300 may also include an exterior layer of loop material such that when the upper straps 3312, 3314 and the lower straps 3316. 3318 are looped back on themselves the hook portions 3320 can connect to parts of the back portion. For example, the crown strap 3348, the left crown piece 3306, the right crown piece 3308. and the neck piece 3310 may include an exterior layer of loop material so that the hook portions 3320 may connect at the respective junctions between the back portion 3302 components and the upper straps 3312, 3314 and the lower straps 3316, 3318.

Also, Fig. 3 shows how the hook portions 3320 may be relatively narrower than their respective straps, as well as the components of the back portion 3302. This may be advantageous in that the straps and the back portion may shield the user's skin from contact with the hook portions thereby minimizing irritation and discomfort. Furthermore. the components of the exemplary positioning and stabilising structure 3300 may be connected to one another to assemble the positioning and stabilising structure by ultrasonic welding. These features may be equally applicable to the different sizes of positioning and stabilising structures discussed below.

### 6.3.1.2.1 Varying Proportions for Different Size Positioning and Stabilising Structures

Figs. 4a-4z1, 5a-5l, and 6a-6v show similar positioning and stabilising structures as well as the individual components of the positioning and stabilising structures. Each series of drawings shows a positioning and stabilising structure of a different size. Thus, Figs. 4a-4z1 show an exemplary positioning and stabilising structure 3340 and its individual components that are dimensioned to accommodate a standard or medium size head. Figs. 5a-5l show an exemplary positioning and stabilising structure 3350 and its individual components that are dimensioned to acconunodate a large size head. In other words, the patient wearing the positioning and stabilising structure 3350 in these views will have a larger head than the patient wearing the positioning and stabilising structure 3340 in Figs. 4a-4z1. Figs. 6a-6v show an exemplary positioning and stabilising structure 3360 and its individual components that are dimensioned to accommodate a small size head. In other words, the patient wearing the positioning and stabilising structure 3360 in these views will have a smaller head than the patient wearing the positioning and stabilising structure 3340 in Figs. 4a-4z1.

### 6.3.1.2.1.1 Standard Size

Figs. 4a-4z1 depict an example of a positioning and stabilising structure 3340 according to the present technology. The exemplary positioning and stabilising structure 3340 shown in these Figures may include a back portion 3340.1. The exemplary back portion 3340.1 may include a left crown piece 3345 and a right crown piece 3342. as well as a neck piece 3343. The positioning and stabilising structure 3340 may also include a crown strap 3348. Also. extending from the back portion 3340.1. may be an upper left strap 3346 and an upper right strap 3341, as well as a lower left strap 3344 and a lower right strap 3344. It should be understood that the lower straps of the exemplary positioning and stabilising structure 3340 may be identical. A hook portion 3349 of hook material may be attached at a distal end of each of the upper straps 3346. 3341 and lower straps 3344. The hook portion 3349 may be used to attach each strap to itself by looping through attachment points of the patient interface. The connection with the hook portions 3349 may be facilitated by a layer of loop material on the exterior of some or all of the components of the positioning and stabilising structure 3340. According to one example of the present technology, the upper straps 3346, 3341 and lower straps 3344 may each have an exterior layer of hook material for releasable attachment with the respective hook portions 3349.

According to another example of the present technology, each component of the positioning and stabilising structure 3340 may also include an exterior layer of loop material such that when the upper straps 3346. 3341 and the lower straps 3344 are looped back on themselves the hook portions 3349 can connect to parts of the back portion. For example. the crown strap 3348, the left crown piece 3345. the right crown piece 3342. and the neck piece 3343 may include an exterior layer of loop material so that the hook portions 3349 may connect at the respective junctions between the back portion 3340.1 components and the upper straps 3346, 3341 and the lower straps 3344.

Also, as discussed above, the hook portions 3349 may be relatively narrower than their respective straps, as well as the components of the back portion 3340.1. Furthermore. the components of the exemplary positioning and stabilising structure 3340 may be connected to one another to assemble the positioning and stabilising structure by ultrasonic welding. For example. each hook portion 3349 may be attached to a respective upper or lower strap by ultrasonic welding at a hook joint 3347. Also, the upper and lower straps may be joined to the components of the back portion by ultrasonic welding at the strap joints 3347.1. The strap joints 3347.1 may be formed, according a further example, by a two-way ultrasonic weld.

It should also be understood that Fig. 4a shows a view of the exemplary positioning and stabilising structure 3340 wherein it is laid out flat and the crown strap 3348 is not connected to the upper left strap 3346. When the crown strap 3348 is connected to the upper left strap 3346, the positioning and stabilising structure 3340 will take on a more curved shape, such as that shown in Fig. 3.

Components of the exemplary positioning and stabilising structure 3340 may be formed by a knitting process. To provide the desired strength and stretchability of these parts the weft of the component used in knitting the component may be oriented in a desired direction. For example, a neck piece weft direction 3343.1 is shown on the neck piece 3343 in Fig. 4h. In Fig. 4n. the right crown piece 3342 and a right crown piece weft direction 3342.1 are shown. In Fig. 4q, the left crown piece 3342 and a left crown piece well direction 3342.1 are shown. In Fig. 4x. the crown strap 3348 and a crown strap weft direction 3348.1 are shown.

Dimensions of the components of the positioning and stabilising structure may vary or remain unchanged between different sizes of positioning and stabilising structures. Fig. 4j shows that the lower right and left straps 3344 may have a length of L₁. which may be about 270.0 mm ± 0.5 mm according to one example. Figs. 4l and 4m depict dimensions L₂, L₃, L₄, and L₅ of the right crown strap 3342. According to one example. L₂ may be about 69.6 mm. L₃ may be about 43.6 mm. L₄ may be about 20.4 mm. and L₅ may be about 67.3 mm. Figs. 4o and 4p depict dimensions L₆, L₇, L₈, and L₉ of the left crown strap 3345, which may be equal to the dimensions L₂, L₃, L₄, and L₅, respectively, of the right crown strap 3342. Figs. 4r and 4s depict dimensions L₁₀, L₁₁, and L₁₂ of the upper right strap 3341. According to one example. L₁₀ may be about 140.0 mm, L₁₁ may be about 155.1 mm, and L₁₂ may be about 40.9 mm. Figs. 4t and 4u depict dimensions L₁₃. L₁₄, and L₁₅ of the upper left strap 3346. which may be equal to the dimensions L₁₀. L₁₁. and L₁₂, respectively. of the upper right strap 3341. Fig. 4w depicts dimension L₁₆ of the crown strap 3348, which may be about 239.7 mm, according to one example.

### 6.3.1.2.1.2 Large Size

Figs. 5a-5l depict an example of a positioning and **stabilising** structure 3350 according to the present technology. The exemplary positioning and stabilising structure 3350 shown in these Figures may include a back portion 3350.1. The exemplary back portion 3350.1 may include a left crown piece 3355 and a right crown piece 3352. as well as a neck piece 3353. The positioning and stabilising structure 3350 may also include a crown strap 3358. Also, extending from the back portion 3350.1 may be an upper left strap 3356 and an upper right strap 3351. as well as a lower left strap 3354 and a lower right strap 3354. It should be understood that the lower straps of the exemplary positioning and **stabilising** structure 3350 may be identical. A hook portion 3359 of hook material may be attached at a distal end of each of the upper straps 3356. 3351 and lower straps 3354. The hook portion 3359 may be used to attach each strap to itself by looping through attachment points of the patient interface. The connection with the hook portions 3359 may be facilitated by a layer of loop material on the exterior of some or all of the components of the positioning and stabilising structure 3350. According to one example of the present technology, the upper straps 3356, 3351 and lower straps 3354 may each have an exterior layer of hook material for releasable attachment with the respective hook portions 3359.

According to another example of the present technology, each component of the positioning and stabilising structure 3350 may also include an exterior layer of loop material such that when the upper straps 3356. 3351 and the lower straps 3354 are looped back on themselves the hook portions 3359 can connect to parts of the back portion. For example, the crown strap 3358, the left crown piece 3355. the right crown piece 3352, and the neck piece 3353 may include an exterior layer of loop material so that the hook portions 3359 may connect at the respective junctions between the back portion 3350.1 components and the upper straps 3356, 3351 and the lower straps 3354.

Also, as discussed above, the hook portions 3359 may be relatively narrower than their respective straps, as well as the components of the back portion 3350.1. Furthermore, the components of the exemplary positioning and stabilising structure 3350 may be connected to one another to assemble the positioning and stabilising structure by ultrasonic welding. For example, each hook portion 3359 may be attached to a respective upper or lower strap by ultrasonic welding at a hook joint 3357. Also, the upper and lower straps may be joined to the components of the back portion by ultrasonic welding of the strap joints 3357.1. The strap joints 3357.1 may be formed, according a further example, by a two-way ultrasonic weld.

It should also be understood that Fig. 5a shows a view of the exemplary positioning and stabilising structure 3350 wherein it is laid out flat and the crown strap 3358 is not connected to the upper left strap 3356. When the crown strap 3358 is connected to the upper left strap 3356, the positioning and stabilising structure 3350 will take on a more curved shape, such as that shown in Fig. 3.

Dimensions of the components of the positioning and stabilising structure may vary or remain unchanged between different sizes of positioning and stabilising structures. Figs. 5f and 5g depict dimensions L₁₇, L₁₈, and L₁₉ of the upper right strap 3351. According to one example, L₁₇ may be about 180.0 mm, L₁₈ may be about 195.1 mm. and L₁₉ may be about 40.9 mm. Figs. 5h and 5i depict dimensions L₂₀, L₂₁, and L₂₂ of the upper left strap 3356, which may be equal to the dimensions L₁₇, L₁₈, and L₁₉, respectively, of the upper right strap 3351. Fig. 5k depicts dimension L₂₃ of the crown strap 3358. which may be about 249.7 mm, according to one example.

As discussed above, some dimensions of the components of the exemplary large size positioning and stabilising structure 3350 may vary as compared to the standard size positioning and stabilising structure 3340 while others may remain the same. In accordance with an example of the present technology, the large size positioning and stabilising structure 3350 may include larger upper right and left straps 3351, 3356 and a larger crown strap 3358 as compared to the corresponding components of the standard size positioning and stabilising structure 3340. Reference may be had to the exemplary dimensions outlined above for details on the differences. Some dimensions may, however, remain unchanged. For example, the large size positioning and stabilising structure 3350 may include the same dimensions as the standard size positioning and stabilising structure 3340 for the neck piece 3353, the lower right and left straps 3354, and the right and left crown pieces 3352. 3355.

Choosing to vary or not to vary certain dimensions of these components for various positioning and stabilising structure sizes may be based on typical patient head size measurements. For example, it may be the case that the regions of the head to be accommodated by the lower straps may not vary between medium and large head sizes while the regions of the head to be accommodated by the upper straps and the crown strap may require longer straps for larger heads.

### 6.3.1.2.1.3 Small Size

Figs. 6a-6l depict an example of a positioning and stabilising structure 3360 according to the present technology. The exemplary positioning and stabilising structure 3360 shown in these Figures may include a back portion 3360.1. The exemplary back portion 3360.1 may include a left crown piece 3365 and a right crown piece 3362, as well as a neck piece 3363. The positioning and stabilising structure 3360 may also include a crown strap 3368. Also, extending from the back portion 3360.1 may be an upper left strap 3366 and an upper right strap 3361, as well as a lower left strap 3364 and a lower right strap 3364. It should be understood that the lower straps of the exemplary positioning and stabilising structure 3360 may be identical. A hook portion 3369 of hook material may be attached at a distal end of each of the upper straps 3366, 3361 and lower straps 3364. The hook portion 3369 may be used to attach each strap to itself by looping through attachment points of the patient interface. The connection with the hook portions 3369 may be facilitated by a layer of loop material on the exterior of some or all of the components of the positioning and stabilising structure 3360. According to one example of the present technology, the upper straps 3366, 3361 and lower straps 3364 may each have an exterior layer of hook material for releasable attachment with the respective hook portions 3369.

According to another example of the present technology, each component of the positioning and stabilising structure 3360 may also include an exterior layer of loop material such that when the upper straps 3366, 3361 and the lower straps 3364 are looped back on themselves the hook ponions 3369 can connect to parts of the back portion. For example, the crown strap 3368, the left crown piece 3365, the right crown piece 3362, and the neck piece 3363 may include an exterior layer of loop material so that the hook portions 3369 may connect at the respective junctions between the back portion 3360.1 components and the upper straps 3366. 3361 and the lower straps 3364.

Also, as discussed above, the hook portions 3369 may be relatively narrower than their respective straps, as well as the components of the back portion 3360.1. Furthermore, the components of the exemplary positioning and stabilising structure 3360 may be connected to one another to assemble the positioning and stabilising structure by ultrasonic welding. For example, each hook portion 3369 may be attached to a respective upper or lower strap by ultrasonic welding at a hook joint 3367. Also, the upper and lower straps may be joined to the components of the back portion by ultrasonic welding at the strap joints 3367.1. The strap joints 3367.1 may be formed, according a further example, by a two-way ultrasonic weld.

It should also be understood that Fig. 6a shows a view of the exemplary positioning and stabilising structure 3360 wherein it is laid out flat and the crown strap 3368 is not connected to the upper left strap 3366. When the crown strap 3368 is connected to the upper left strap 3366, the positioning and stabilising structure 3360 will take on a more curved shape, such as that shown in Fig. 3.

Dimensions of the components of the positioning and stabilising structure may vary or remain unchanged between different sizes of positioning and stabilising structures. Fig. 6h shows that the lower right and left straps 3364 may have a length of L₂₄, which may be about 230.0 mm ± 0.5 mm according to one example. Figs. 6j and 6k depict dimensions L₂₅, L₂₆, L₂₇, and L₂₈ of the right crown strap 3362. According to one example. L₂₅ may be about 64.6 mm, L₂₆ may be about 38.6 mm, L₂₇ may be about 20.9 mm, and L₂₈ may be about 63.6 mm. Figs. 6m and 6n depict dimensions L₂₉, L₃₀, L₃₁, and L₃₂ of the left crown strap 3365. which may be equal to the dimensions L₂₅, L₂₆, L₂₇, and L₂₈, respectively, of the right crown strap 3362. Figs. 6p and 6q depict dimensions L₃₃, L₃₄, and L₃₅ of the upper right strap 3361. According to one example. L₃₃ may be about 130.0 mm, L₃₄ may be about 145.8 mm, and L₃₅ may be about 41.9 mm. Figs. 6r and 6s depict dimensions L₃₆, L₃₇, and L₃₈ of the upper left strap 3366, which may be equal to the dimensions L₃₃, L₃₄, and L₃₅, respectively, of the upper right strap 3361. Fig. 6t depicts dimension L₃₉ of the crown strap 3368, which may be about 227.4 mm, according to one example.

As discussed above, some dimensions of the components of the exemplary small size positioning and stabilising structure 3360 may vary as compared to the standard size positioning and stabilising structure 3340 while others may remain the same. According to an example of the present technology, the small size headgear 3360 may only use a neck piece 3363 of the same dimensions as the standard size positioning and stabilising structure 3340. The remaining components may be sized according to the dimensions disclosed above.

Choosing to vary or not to vary certain dimensions of these components for various positioning and stabilising structure sizes may be based on typical patient head size measurements. In contrast to the large and standard size positioning and stabilising structures that may share several dimensions, it may be the case that as the patient's head decreases in size that more components of the positioning and stabilising structure may need to be decreased to accommodate the smaller head size.

### 6.3.2 Evenly Distributed Sealing Force

According to an example of the present technology, the positioning and stabilising structure 3300 may generate an even distribution of sealing force on the seal-forming structure to seal against the face of the patient.

### 6.3.2.1 Sealing Force Vectors and the Frankfort Horizontal

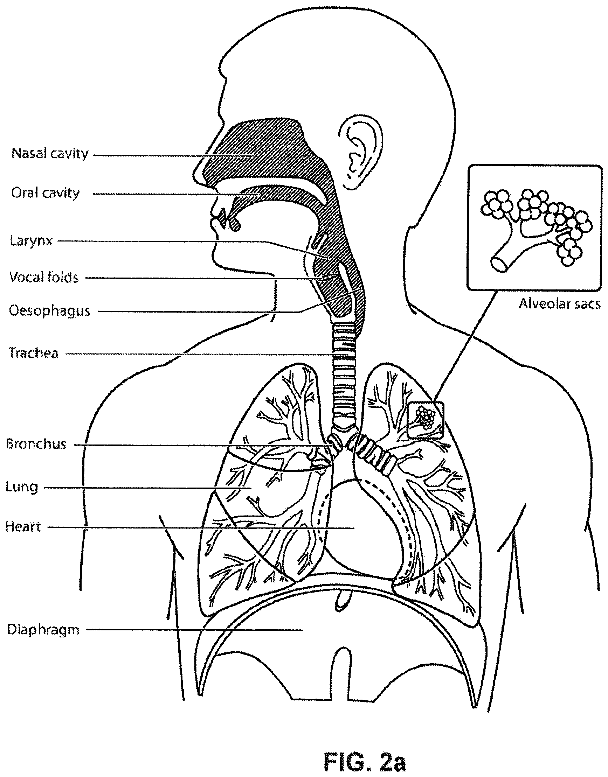

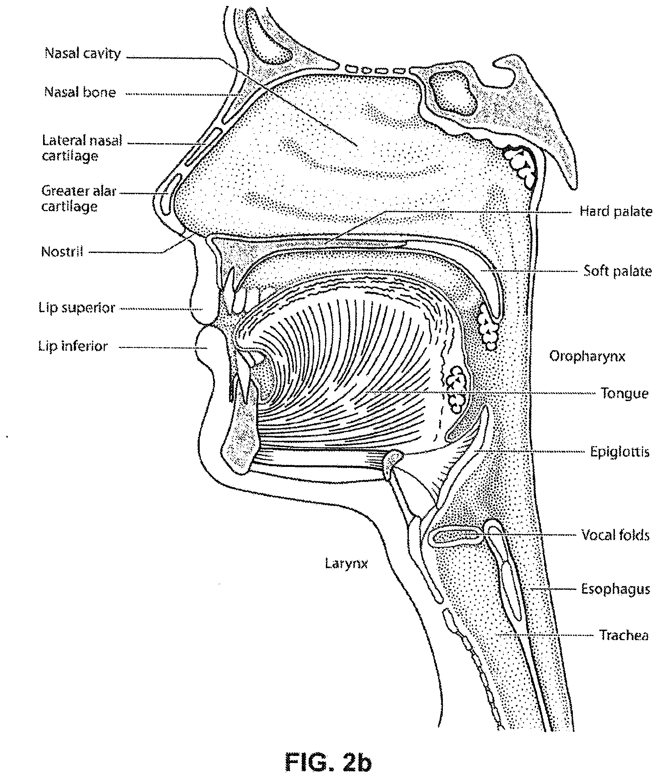

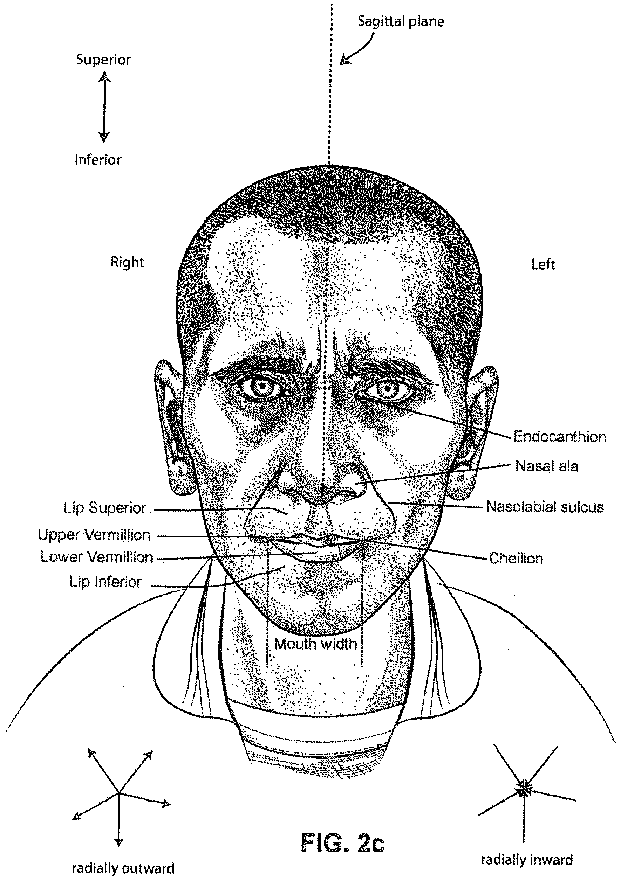

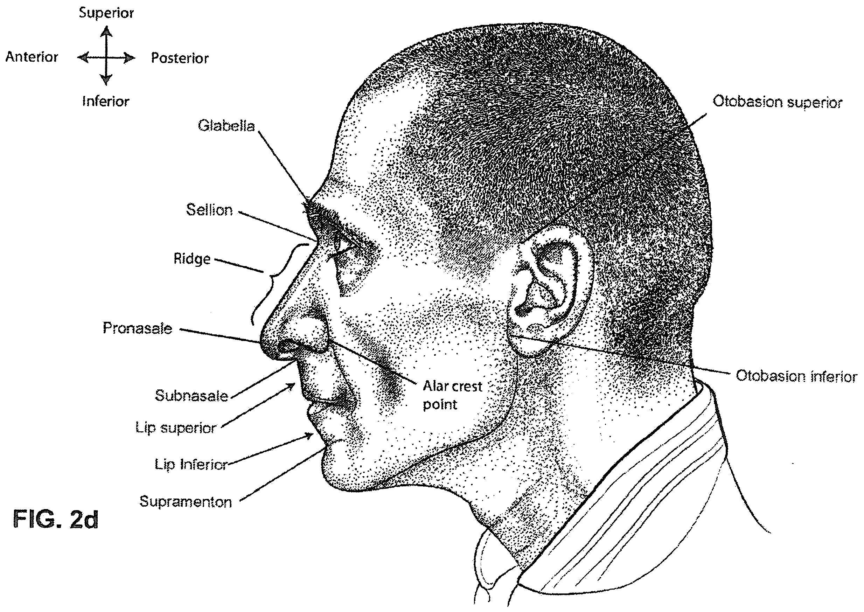

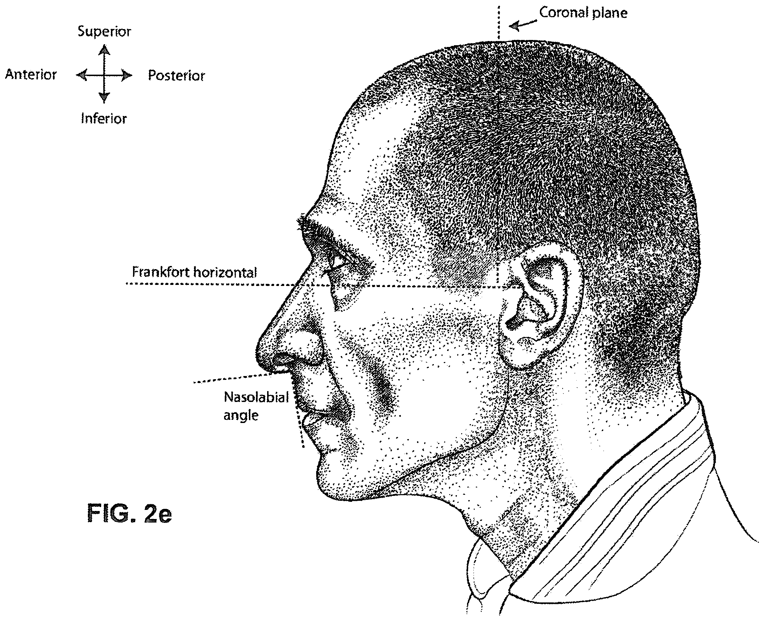

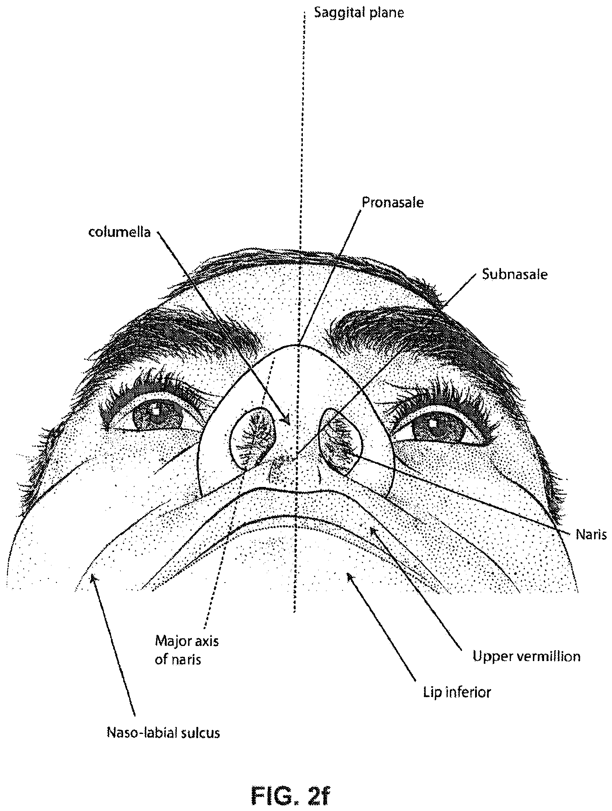

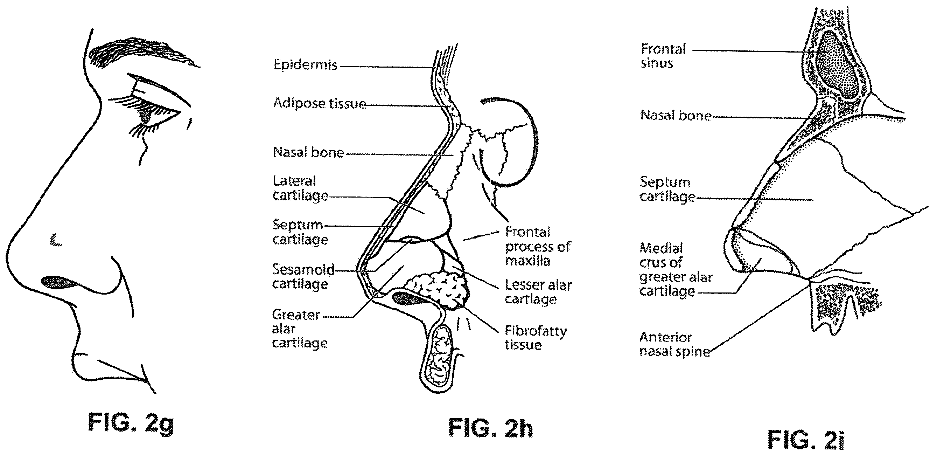

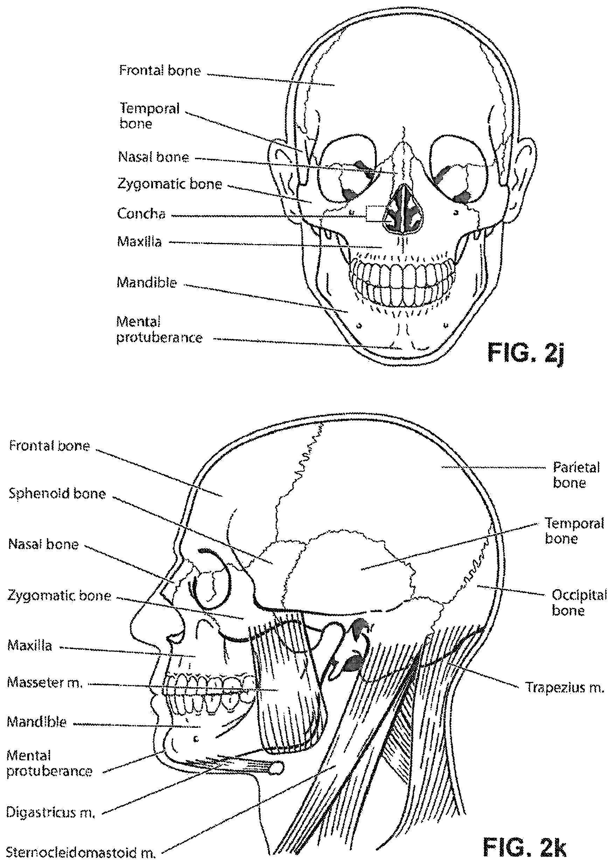

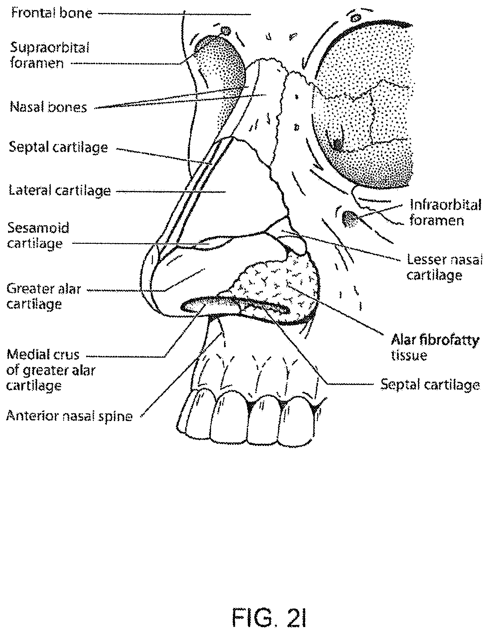

In Fig. 8c. an upper sealing force vector F₁ is shown being generated by the upper right strap 3314 and a lower scaling force vector F₂ is shown being generated by the lower right strap 3318. According to an example of the present technology, both sealing force vectors F₁ and F₂ (and their counterparts not shown on the other side of the patient's head) are intended to be directed substantially parallel to the Frankfort horizontal which can be seen in Fig. 2e. By maintaining these sealing force vectors in a substantially parallel orientation relative to the Frankfort horizontal it may be possible to more evenly distribute the sealing force of the seal-forming structure 3100 against the face of the patient. This may improve patient comfort because there is no location along the seal-forming structure 3100 that presses against the face with an amount of force in excess of another location. In accordance with another example of the present technology, the sealing force vectors generated by upper and lower straps may be substantially parallel to one another.

### 6.3.2.2 Connection of the Patient Interface Frame and the Cushion Assembly

Figs. 7a-7e, 7j-7m, 7o, 7p, and 14-18 show various views of a patient interface frame according to an example of the present technology. Figs. 8a-8c show various views of a patient interface 3000 and a positioning and stabilising structure 3300 assembled and donned on a patient. As discussed above, the seal-forming structure 3100 may be a silicone component that is molded onto the plenum chamber 3200 that may be made of Nylon to form a cushion assembly. Nylon 12 may also be used to form the plenum chamber 3200 in a further example of the technology. In another example of the technology polycarbonate may be used to form the plenum chamber 3200. A patient interface frame 3370 facilitates the connection between the cushion assembly and the positioning and stabilising structure 3300. so it must be connectable to the cushion assembly to transfer the sealing forces from the positioning and stabilising structure. The plenum chamber 3200 and the patient interface frame 3370 may include cooperating features to allow these components to be friction fit to one another. The exemplary patient interface frame 3370 may include a pair of upper patient interface frame retention features 3384 and a pair of lower patient interface frame retention features 3382. The plenum chamber 3200 may also include a corresponding pair of upper plenum chamber retention features and a corresponding pair of lower plenum chamber features, although these are not shown in these views. As can be seen in these views, the upper and lower patient interface frame retention features 3384, 3382 may be formed as protrusions from the patient interface frame 3370. Thus, the plenum chamber's 3200 upper and lower plenum chamber retention features would be corresponding indentations or receptacles to affect the friction fit. An opposite configuration is also envisioned.

As can be seen in these views, the patient interface frame 3370 may be shaped to surround a periphery of the plenum chamber 3200. If the plenum chamber 3200 is made from polycarbonate, which is a transparent material, this may allow the bed partner to see more of the facial features of the patient, thereby making the patient interface 3000 more pleasant looking. It should, therefore, be understood that the connection between the plenum chamber 3200 and the patient interface frame 3370 may be characterized as hard-to-hard.

Also, by connecting the patient interface frame 3370 to the plenum chamber 3200 about its periphery a more even transfer of scaling force to the seal-forming structure 3100 may be facilitated. According to an example of the present technology, the plenum chamber 3200 may be formed from polycarbonate or another relatively rigid material, while the seal-forming structure 3100 may be formed from a relatively pliable material such as silicone. Tension in the positioning and stabilising structure 3300 is transferred via the patient interface frame 3370 to the plenum chamber 3200 about the periphery of the plenum chamber. The seal-forming structure 3100 may also be molded to the plenum chamber 3200 about its periphery. Thus, an even distribution of sealing force about the periphery of the plenum chamber 3200 may lead to an even distribution of that force to the seal-forming structure 3100 as it is also connected to the plenum chamber about its periphery.

An additional concept that should be understood in the selection of materials of the plenum chamber 3200 and the seal-forming structure 3100 is that minimal deformation is desired for the plenum chamber while some deformation intended for the seal-forming structure. Thus, for the plenum chamber 3200 a material should be chosen such that the plenum chamber will not deform under the tension loads generated by the straps. The seal-forming structure 3100 on the other hand may be intended to deform to form a seal around the facial features of the patient. Thus, the seal-forming structure 3100 should be formed from a material that will deform sufficiently to affect a pneumatic seal while not deforming to such a degree that the plenum chamber 3200 presses against the face. By selecting materials based on this concept it may be possible to ensure that an even distribution of sealing force is provided about the periphery of the seal-forming structure 3100 to maximize patient comfort.

Figs. 19-21 show another example of the present technology where the seal-forming structure 3100 may be detachable from the plenum chamber 3200. Fig. 19 shows the plenum chamber 3200 with a first attachment region 3202 that may extend around the periphery of the plenum chamber 3200 to join with the seal-forming structure 3100. The plenum chamber 3200 may also include upper receivers 3208 to receive the upper patient interface frame retention features 3384 and lower receivers 3206 to receive the lower patient interface frame retention features 3382. An upper receiver 3208 and a lower receiver 3206 may, accordingly, be provided on each side of the plenum chamber 3200 to receive corresponding ones of the upper patient interface frame retention features 3384 and the lower patient interface frame retention features 3382 when the patient interface frame 3370 is attached. Detents 3204 may also be provided to the plenum chamber 3200 to facilitate a snap-fit connection with the seal-forming structure 3100. A detents 3204 may be provided on each side of the plenum chamber 3200.

Fig. 20 shows the seal-forming structure 3100 according to the present example. The seal-forming structure 3100 may include a second attachment region 3102 to attach the seal-forming structure 3100 to the plenum chamber 3200 at the first attachment region. The second attachment region 3102 may be formed around the periphery of the seal-forming structure 3100. The first attachment region 3202 and the second attachment region 3102 may be shaped to conform to one another in a press-fit, snap-fit, and/or friction-fit. One of the first attachment region 3202 and the second attachment region 3102 may be formed from a more compliant and/or flexible material than the other to allow for a secure fit by deforming to provide a conforming attachment. Also, the seal-forming structure 3100 may be provided with protrusions 3104 corresponding to the detents 3204 of the plenum chamber to form a snap-fit therewith and secure the seal-forming structure 3100 to plenum chamber 3200.

Fig. 21 shows the present example with the seal-fonning structure 3100 and the plenum chamber 3200 joined to one another at their respective peripheries by engagement of the first attachment region 3202 and the second attachment region 3102.

Further examples of attachment between a seal-forming structure 3100 and a plenum chamber 3200 that are envisioned are disclosed in US Patent Nos. 6.491.034, 6,412.487, 6,823,869.

### 6.3.2.3 Rigidiser Arms

According to an example of the present technology, the patient interface frame 3370 may include a pair of rigidiser arms 3371, 3381. Figs. 7f and 7g depict the right rigidiser arm 3371. The right rigidiser arm 3371 may include an upper right attachment point 3375 for attachment to the upper right strap 3314 and a right rigidiser arm connection feature 3373 for connection to a right frame connection feature 3376 of the patient interface frame 3370. Figs. 7h and 7i depict the left rigidiser arm 3381. The left rigidiser ann 3381 may include an upper left attachment point 3385 for attachment to the upper left strap 3312 and a left rigidiser arm connection feature 3383 for connection to a left frame connection feature 3378 of the patient interface frame 3370. Each rigidiser arm connection feature 3373, 3383 may connect its respective rigidiser arm 3371, 3381 to the patient interface frame 3370 by being directly connected to and/or engaged with a respective frame connection feature 3376. 3378. The rigidiser arms 3371. 3381 may be formed from polycarbonate and/or nylon. According to another example of the technology, flexible connection structures 3377. 3387 may connect the rigidiser arm connection features 3373, 3383 to the respective frame connection features 3376. 3378 without these connection features being in direct connection and/or engagement such that the flexible connection structures serve as intermediate connection pieces.

As can be seen in Figs. 8a-8c, the rigidiser arms 3371, 3381 may be shaped such that when the patient interface 3000 and the positioning and stabilising structure 3300 are donned by the patient the rigidiser arms do not pass over the eyes. The rigidiser arms 3371, 3381 may also be shaped to avoid the ears and the temples. As can be seen in Fig. 8c, the right rigidiser arm 3371 is shaped to pass along and conform to the patient's cheek and extend between the eye and car while avoiding the temple such that the upper right strap 3314 connects to the right rigidiser ann at the upper right attachment point 3375 above the ear.

Also, as can be seen from these views, the rigidiser arms 3371, 3381 are relatively wider along their longitudinal axes. By forming the rigidiser arms 3371. 3381 this may allow for targeted flexing of the rigidiser arms. In other words, flexing is resisted more greatly in the direction of the wider cross-section while it is resisted less in the direction of the narrower cross-section. This may be advantageous in that the rigidiser arms 3371, 3381 may be allowed to flex toward the patient's face and cheeks in conformity therewith when placed in tension by the positioning and stabilising structure, while flexing upward into the patient's eyes or downward toward the patient's ears is resisted.

Figs. 8a-8c also show an attachment point plane PA and a connection point plane PC offset vertically from one another by a distance O. Both planes should be understood to be substantially parallel to the Frankfort horizontal depicted in Fig. 2e. Also, the attachment point plane PA is located such that the upper attachment points 3375, 3385 of the rigidiser arms 3371, 3381 are located within the attachment point plane. Furthermore, the connection point plane PC is located such that right and left connection points 3391, 3393 between the rigidiser arms 3371. 3381 and the patient interface frame 3370 lie within the connection point plane.

Figs. 10a and 10b show exemplary desired rigidiser arm paths along a side profile of a patient's head for large and small size beads, respectively. The desired rigidiser arm paths are within the rectangular regions having widths of 68mm and 51mm, respectively. The measurements indicated in these drawings represent the basis for sizing and shaping the rigidiser arms such that they follow the indicated paths so as to provide optimal patient comfort.

### 6.3.2.3.1 Attachment Points for the Positioning and Stabilising Structure

According to an example of the present technology, the rigidiser arms 3371, 3381 may provide upper attachments points 3375, 3385 for the upper straps 3312, 3314 of the positioning and stabilising structure 3300, as discussed above. According to an example of the present technology the upper attachment points 3375, 3385 may be slots formed at respective ends of the rigidiser arms 3371, 3381. The upper straps 3312, 3314 may be connected to the respective upper attachment points 3375, 3385 by looping through them. The patient interface frame 3370 may also include lower attachment points 3372. 3374 for the lower straps 3316, 3318, which can be seen in Figs. 7a-7c. 7e, 7j. 7k. 7p, and 8a-8c. The lower attachment points 3372, 3374 may each include a hook 3380 as shown in Figs. 7j-7n The hook 3380 may help retain the respective lower strap 3316, 3318 when it is looped around the lower attachment point, as shown in Figs. 8a-8c. As discussed above, forming the lower attachment points 3372. 3374 as depicted would allow the patient to leave the hook portions 3320 connected to the respective lower straps 3316, 3318 when doffing the patient interface 3000 and the positioning and stabilising structure 3300. The patient may be able to simply slip the looped portion formed by attaching the hook portions 3320 to the respective lower straps 3316, 3318 over the respective lower attachment points 3372, 3374 when donning the patient interface 3000 and the positioning and stabilising structure 3300. The lower straps 3316, 3318 would then be retained by the hooks 3380.

Fig. 13 shows another example of a rigidiser arm 3371 according to the present technology. The rigidiser arm 3371 shown in this view may include a rigidiser arm connection feature 3373. This exemplary rigidiser arm 3371 may also be shaped and dimensioned similarly to the rigidiser arm shown in Figs. 7f and 7g. The upper attachment point 3375 may be different, however, in that it may include an opening 3379 into the slot that comprises the attachment point. By shaping the attachment point 3375 as depicted the patient may be able to pull the looped upper straps from the upper attachment points without disconnecting the hook portions from the respective upper straps. This may be advantageous for the patient because the patient would not be required to adjust the length of the upper straps each time the patient interface 3000 and the positioning and stabilising structure 3300 are donned. Rather, the patient would be able to simply slip the upper straps out of the attachment points of the respective rigidiser arms. Also, in this example the opening may narrow into the attachment point 3375. This may make it easier to slide the straps into the attachment points but make it more difficult to remove the straps and, therefore, less likely that the straps will fall off during therapy.

It is also envisioned that other attachment configurations may be provided for connecting the straps to the attachment points. For example, clips may be provided, through which the straps loop, and the clips in turn may attach to respective attachment points (i.e., receptacles) of the rigidiser arms and the patient interface frame.

### 6.3.2.4 Connection of the Rigidiser Arms and the Patient Interface Frame

According to an example of the present technology, the rigidiser arms 3371. 3381 may be connected to the patient interface frame 3370 in a hinge-like manner. The hinge may be mechanical or living. A mechanical hinge may be formed by the patient interface frame 3370 having a post about which the rigidiser arms 3371. 3381 are connected and rotate. A living hinge may involve a flexible connection that is discussed in greater detail below. The hinge in an example of the technology may comprise a flexible silicone hinge. Regardless of the type of hinge used, it may be advantageous to form the hinge such that the rigidiser arms 3371, 3381 are rotatable substantially in a single plane that is substantially parallel to the Frankfort horizontal shown in Fig. 2e. This may help to keep the rigidiser arms 3371, 3381 from rotating upward and into the patient's field of view or downward and against the ears.

It should be understood that the patient interface frame 3370 and the rigidiser anns 3371. 3381 may be formed separately and then connected by a mechanical connection, either permanently or releasably. Alternatively, these components may be joined by overmolding one to the other. In a further alternative, the patient interface frame 3370 and the rigidiser anns 3371, 3381 may be formed separately and then connected by overmolding a third component over the connection to facilitate the connection. In a still further alternative, the patient interface frame 3370 and the rigidiser arms 3371, 3381 may be molded together as one piece and in a further variation an additional component may be overmolded to the joint to further control the flexibility of the rigidiser arms.

To facilitate a connection between the rigidiser arms 3371, 3381 and the patient interface frame 3370 with a desired level of flexibility, flexible connection structures 3377, 3387 may connect the right rigidiser arm connection feature 3373 and the right frame connection feature 3376 and the left rigidiser arm connection feature 3383 and the left frame connection feature 3378. The flexible connection structures 3377, 3387 may be formed from a material such as silicone or a thermoplastic elastomer that is relatively more flexible than the rigidiser arms 3371, 3381 and the patient interface frame 3370. The flexible connection structures 3377, 3387 may also be overmolded onto the rigidiser arms 3371, 3381 and the patient interface frame 3370 to facilitate a permanent connection between these components.

According to another example of the present technology, shown in Figs. 22 and 23, the connection points for the positioning and stabilising structure 3300 may be formed directly on or integrally with the plenum chamber 3200. According to this example, the right frame connection feature 3376 and the left frame connection feature 3378 may be formed integrally with the plenum chamber 3200. The flexible connection structures 3377, 3387 may be overmolded directly onto the plenum chamber 3200 at the right frame connection feature 3376 and the left frame connection feature 3378, respectively, to attach the rigidiser arms 3371, 3381. Also, the lower attachment points 3372, 3374 may be formed integrally with the plenum chamber 3200. It should be understood that in such an example that the patient interface frame 3370 may be unnecessary as the flexible connection structures 3377. 3387 and the lower attachment points 3372, 3374 are integral with the plenum chamber 3200. Accordingly, the seal-forming structure 3100 may be joined to or formed integrally with the plenum chamber 3200. This example may be advantageous in that fewer components are required, which may reduce manufacturing costs and provide a simpler patient interface system for the patient.

### 6.3.3 Improved Stability of the Positioning and Stabilising Structure

According to an example of the present technology, the positioning and stabilising structure 3300 may provide improved stability, particularly in the region of the crown of the head. Improvements in stability of the positioning and stabilising structure 3300 over previous positioning and stabilising structures may be accomplished by widening components of the positioning and stabilising structure.

### 6.3.3.1 Widened Connection Portion

According to an example of the present technology, a connection region of the positioning and stabilising structure 3300 near the crown of the head and at the connection between the upper straps 3316, 3318. the crown strap 3304, and the crown pieces 3306, 3308 may be widened to improve stability. This may be done by widening the aforementioned components near this region. It may be desirable to widen the above-mentioned portions of the exemplary positioning and stabilising structure 3300 to better counteract riding up of the patient interface 3000, which may occur due to displacement of the positioning and stabilising structure from its intended position. Increasing the surface area these regions of connection may enhance stiffness so that the back portion 3302 remains on the back of the head. It may also result in better resistance to buckling in the straps. Stability may also be enhanced by widening these components because a larger surface area of the head is contacted.

Fig. 9 shows the standard size positioning and stabilising structure 3340 laid flat with a related art positioning and stabilising structure 3399 that is designed to fit the same size head. The regions of relatively increased width are visible in this view to indicate how much wider these regions are relative to the related art positioning and stabilising structure 3399, which is more prone slipping and buckling.

### 6.3.4 Vent 3400

In one form, the patient interface 3000 includes a vent 3400 constructed and arranged to allow for the washout of exhaled carbon dioxide.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure 3500. e.g. a swivel.

### 6.3.5 Decoupling structure(s) 3500

In one form the patient interface 3000 includes at least one decoupling structure 3500, for example a swivel or a ball and socket

### 6.3.6 Connection port 3600

Connection port 3600 allows for connection to the air circuit 4170.

### 6.3.7 Anti-asphyxia 3800

In one form, the patient interface 3000 includes an anti-asphyxia valve 3800.

### 6.3.8 Ports 3900

In one form of the present technology, a patient interface 3000 includes one or more ports that allow access to the volume within the plenum chamber 3200. In one form this allows a clinician to supply supplemental oxygen. In one form this allows for the direct measurement of a property of gases within the plenum chamber 3200. such as the pressure.

### 6.3.9 Patient Interface and Positioning and Stabilising Structure Assembly

Figs. 11 and 12 depict perspective view of exemplary patient interfaces 3000 and positioning and stabilising structures 3300. Both exemplary devices depict similar features inclusive of those discussed above.

### 6.4 GLOSSARY

For the purposes of the present technology disclosure, in certain forms of the present technology, one or more of the following definitions may apply. In other forms of the present technology, alternative definitions may apply.

### 6.4.1 General

*Air*: In certain forms of the present technology, air supplied to a patient may be atmospheric air, and in other forms of the present technology atmospheric air may be supplemented with oxygen.

*Continuous Positive Airway Pressure (CPAP)*: CPAP treatment will be taken to mean the application of a supply of air or breathable gas to the entrance to the airways at a pressure that is continuously positive with respect to atmosphere, and preferably approximately constant through a respiratory cycle of a patient. In some forms, the pressure at the entrance to the airways will vary by a few centimeters of water within a single respiratory cycle, for example being higher during inhalation and lower during exhalation. In some forms, the pressure at the entrance to the airways will be slightly higher during exhalation, and slightly lower during inhalation. In some forms, the pressure will vary between different respiratory cycles of the patient, for example being increased in response to detection of indications of partial upper airway obstruction, and decreased in the absence of indications of partial upper airway obstruction.

### 6.4.2 Aspects of PAP devices

*Air circuit*: A conduit or tube constructed and arranged in use to deliver a supply of air or breathable gas between a PAP device and a patient interface. In particular, the air circuit may be in fluid connection with the outlet of the pneumatic block and the patient interface. The air circuit may be referred to as air delivery tube. In some cases there may be separate limbs of the circuit for inhalation and exhalation. In other cases a single limb is used.

*APAP*: Automatic Positive Airway Pressure. Positive airway pressure that is continually adjustable between minimum and maximum limits, depending on the presence or absence of indications of SDB events.

*Blower or flow generator*: A device that delivers a flow of air at a pressure above ambient pressure.

*Controller*: A device, or portion of a device that adjusts an output based on an input. For example one form of controller has a variable that is under control- the control variable- that constitutes the input to the device. The output of the device is a function of the current value of the control variable, and a set point for the variable. A servo-ventilator may include a controller that has ventilation as an input, a target ventilation as the set point, and level of pressure support as an output. Other forms of input may be one or more of oxygen saturation (SaO2), partial pressure of carbon dioxide (PCO2), movement, a signal from a photoplethysmogram, and peak flow. The set point of the controller may be one or more of fixed, variable or learned. For example, the set point in a ventilator may be a long term average of the measured ventilation of a patient. Another ventilator may have a ventilation set point that changes with time. A pressure controller may be configured to control a blower or pump to deliver air at a particular pressure.

*Therapy:* Therapy in the present context may be one or more of positive pressure therapy, oxygen therapy, carbon dioxide therapy, control of dead space, and the administration of a drug.

*Motor*: A device for converting electrical energy into rotary movement of a member. In the present context the rotating member is an impeller, which rotates in place around a fixed axis so as to impart a pressure increase to air moving along the axis of rotation.

*Positive Airway Pressure (PAP) device*: A device for providing a supply of air at positive pressure to the airways.

*Transducers:* A device for converting one form of energy or signal into another. A transducer may be a sensor or detector for converting mechanical energy (such as movement) into an electrical signal. Examples of transducers include pressure sensors, flow sensors, carbon dioxide (CO₂) sensors, oxygen (O₂) sensors, effort sensors, movement sensors, noise sensors, a plethysmograph, and cameras.

*Volute:* The casing of the centrifugal pump that receives the air being pumped by the impeller, slowing down the flow rate of air and increasing the pressure. The cross-section of the volute increases in area towards the discharge port.

### 6.4.3 Aspects of the respiratory cycle

*Apnea*: Preferably, apnea will be said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort.

Breathing rate: The rate of spontaneous respiration of a patient, usually measured in breaths per minute.

Duty cycle: The ratio of inhalation time, *Ti* to total breath time, *Ttot.*

*Effort* (*breathing*): Preferably breathing effort will be said to be the work done by a spontaneously breathing person attempting to breathe.

*Expiratory portion of a breathing cycle:* The period from the start of expiratory flow to the start of inspiratory flow.

*Flow limitation*: Preferably, flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
(i) *Flattened*: Having a rise followed by a relatively flat portion, followed by a fall.
(ii) *M-shaped:* Having two local peaks, one at the leading edge, and one at the trailing edge, and a relatively flat portion between the two peaks.
(iii) *Chair-shaped*: Having a single local peak, the peak being at the leading edge, followed by a relatively flat portion.
(iv) *Reverse-chair shaped*: Having a relatively flat portion followed by single local peak, the peak being at the trailing edge.

*Hypopnea*: Preferably, a hypopnea will be taken to be a reduction in flow, but not a cessation of flow. In one form, a hypopnea may be said to have occurred when there is a reduction in flow below a threshold for a duration. In one form in adults, the following either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 39% or an arousal.

*Hyperpnea*: An increase in flow to a level higher than normal flow?.

*Inspiratory portion of a* breathing *cycle*: Preferably the period from the start of inspiratory flow to the start of expiratory flow will be taken to be the inspiratory portion of a breathing cycle.

*Patency (airway)*: The degree of the airway being open, or the extent to which the airway is open. A *patent* airway is open. Airway patency may be quantified, for example with a value of one (1) being patent, and a value of zero (0), being closed.

*Positive End-Expiratory Pressure (PEEP)*: The pressure above atmosphere in the lungs that exists at the end of expiration.

*Peak flow* (*Qpeak*): The maximum value of flow during the inspiratory portion of the respiratory flow waveform.

*Respiratory flow, airflow, patient airflow, respiratory airflow* (*Qr*): These synonymous terms may be understood to refer to the PAP device's estimate of respiratory airflow, as opposed to "true respiratory flow" or "true respiratory airflow", which is the actual respiratory flow experienced by the patient, usually expressed in litres per minute.

*Tidal volume* (*Vt*): The volume of air inhaled or exhaled during normal breathing, when extra effort is not applied.

*(inhalation) Time* (*Ti*): The duration of the inspiratory portion of the respiratory flow waveform.

*(exhalation) Time* (*Te*): The duration of the expiratory portion of the respiratory flow waveform.

*(total) Time* (*Ttot*): The total duration between the start of the inspiratory portion of one respiratory flow waveform and the start of the inspiratory portion of the following respiratory flow waveform.

*Typical recent ventilation*: The value of ventilation around which recent values over sonic predetennined timescale tend to cluster, that is, a measure of the central tendency of the recent values of ventilation.

*Upper airway obstruction* (*UAO*): includes both partial and total upper airway obstruction. This may be associated with a state of flow limitation, in which the level of flow increases only slightly or may even decrease as the pressure difference across the upper airway increases (Starling resistor behaviour).

*Ventilation* (*Vent*): A measure of the total amount of gas being exchanged by the patient's respiratory system, including both inspiratory and expiratory flow, per unit time. When expressed as a volume per minute, this quantity is often referred to as "minute ventilation". Minute ventilation is sometimes given simply as a volume, understood to be the volume per minute.

### 6.4.4 PAP device parameters

*Flow rate*: The instantaneous volume (or mass) of air delivered per unit time. While flow rate and ventilation have the same dimensions of volume or mass per unit time, flow rate is measured over a much shorter period of time. Flow may be nominally positive for the inspiratory portion of a breathing cycle of a patient, and hence negative for the expiratory portion of the breathing cycle of a patient. In some cases, a reference to flow rate will be a reference to a scalar quantity, namely a quantity having magnitude only. In other cases, a reference to flow rate will be a reference to a vector quantity, namely a quantity having both magnitude and direction. Flow will be given the symbol *Q.* Total flow, *Qt,* is the flow of air leaving the PAP device. Vent flow, *Qv*, is the flow of air leaving a vent to allow washout of exhaled gases. Leak flow, *Ql,* is the flow rate of unintentional leak from a patient interface system. Respiratory flow, *Qr,* is the flow of air that is received into the patient's respiratory system.

*Leak*: Preferably, the word leak will be taken to be a flow of air to the ambient. Leak may be intentional, for example to allow for the washout of exhaled CO₂. Leak may be unintentional, for example, as the result of an incomplete seal between a mask and a patient's face.

*Pressure:* Force per unit area. Pressure may be measured in a range of units, including cmH₂O, g-f/cm², hectopascal. 1cmH₂O is equal to 1 g-f/cm² and is approximately 0.98 hectopascal. In this specification, unless otherwise stated, pressure is given in units of cmH₂O. For nasal CPAP treatment of OSA, a reference to treatment pressure is a reference to a pressure in the range of about 4-20 cmH₂O, or about 4-30 cmH₂O. The pressure in the patient interface is given the symbol *Pm.*

*Sound Power*: The energy per unit time carried by a sound wave. The sound power is proportional to the square of sound pressure multiplied by the area of the wavefront. Sound power is usually given in decibels SWL. that is, decibels relative to a reference power, normally taken as 10⁻¹² watt.

*Sound Pressure:* The local deviation from ambient pressure at a given time instant as a result of a sound wave travelling through a medium. Sound power is usually given in decibels SPL, that is, decibels relative to a reference power, normally taken as 20 × 10⁴ pascal (Pa), considered the threshold of human hearing.

### 6.4.5 Terms for ventilators

*Adaptive Servo-Ventilator*: A ventilator that has a changeable, rather than fixed target ventilation. The changeable target ventilation may be learned from some characteristic of the patient, for example, a respiratory characteristic of the patient.

*Backup rate*: A parameter of a ventilator that establishes the minimum respiration rate (typically in number of breaths per minute) that the ventilator will deliver to the patient, if not otherwise triggered.

*Cycled:* The termination of a ventilator's inspiratory phase. When a ventilator delivers a breath to a spontaneously breathing patient, at the end of the inspiratory portion of the breathing cycle, the ventilator is said to be cycled to stop delivering the breath.

*EPAP* (or *EEP*): a base pressure, to which a pressure varying within the breath is added to produce the desired mask pressure which the ventilator will attempt to achieve at a given time.

*IPAP*: desired mask pressure which the ventilator will attempt to achieve during the inspiratory portion of the breath.

*Pressure support*: A number that is indicative of the increase in pressure during ventilator inspiration over that during ventilator expiration, and generally means the difference in pressure between the maximum value during inspiration and the minimum value during expiration (c.g., *PS = IPAP - EPAP*). In some contexts pressure support means the difference which the ventilator aims to achieve, rather than what it actually achieves.

*Servo-ventilator*: A ventilator that measures patient ventilation has a target ventilation, and which adjusts the level of pressure support to bring the patient ventilation towards the target ventilation.

*Spontaneous*/*Timed (S*/*T)* - A mode of a ventilator or other device that attempts to detect the initiation of a breath of a spontaneously breathing patient. If however, the device is unable to detect a breath within a predetermined period of time. the device will automatically initiate delivery of the breath.

*Swing*: Equivalent term to pressure support.

*Triggered*: When a ventilator delivers a breath of air to a spontancously breathing patient, it is said to be triggered to do so at the initiation of the respiratory portion of the breathing cycle by the patient's efforts.

*Ventilator*: A mechanical device that provides pressure support to a patient to perform some or all of the work of breathing.

### 6.4.6 Anatomy of the face

*Ala*: the external outer wall or "wing" of each nostril (plural: alar)

*Alare*: The most lateral point on the nasal *ala.*

*Alar curvature (or alar crest) point:* The most posterior point in the curved base line of each *ala,* found in the crease formed by the union of the *ala* with the cheek.

*Auricula or Pinna*: The whole external visible part of the ear.

*(nose) Bony framework*: The bony framework of the nose comprises the nasal bones, the frontal process of the maxillae and the nasal part of the frontal bone.

*(nose) Cartilaginous framework*: The cartilaginous framework of the nose comprises the septal, lateral, major and minor cartilages.

*Columella*: the strip of skin that separates the nares and which runs from the *pronasale* to the upper lip.

*Columella angle*: The angle between the line drawn through the midpoint of the nostril aperture and a line drawn perpendicular to the Frankfun horizontal while intersecting subnasale.

*Frankfort horizontal plane*: A line extending from the most inferior point of the orbital margin to the left tragion. The tragion is the deepest point in the notch superior to the tragus of the auricle.

*Glabella*: Located on the soft tissue, the most prominent point in the midsagittal plane of the forehead.

*Lateral nasal cartilage*: A generally triangular plate of cartilage. Its superior margin is attached to the nasal bone and frontal process of the maxilla, and its inferior margin is connected to the greater alar cartilage.

*Greater alar canilage*: A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares* (*Nostrils*): Approximately ellipsoidal apertures forming the entrance to the nasal cavity. The singular form of nares is naris (nostril). The nares are separated by the nasal septum.

*Naso-labial sulcus or Naso-labial fold*: The skin fold or groove that runs from each side of the nose to the corners of the mouth, separating the cheeks from the upper lip.

*Naso-labial angle*: The angle between the columella and the upper lip, while intersecting subnasalc.

*Otobasion inferior*: The lowest point of attachment of the auricle to the skin of the face.

*Orabasion superior*: The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal)*: The nasal ridge is the midline prominence of the nose, extending from the Sellion to the Pronasale.

*Sagittal plane*: A venical plane that passes from anterior (front) to posterior (rear) dividing the body into right and left halves.

*Sellion:* Located on the soft tissue, the most concave point overlying the area of the frontonasal suture.

*Septal cartilage (nasal):* The nasal septal cartilage forms part of the septum and divides the front part of the nasal cavity.

*Subalare*: The point at the lower margin of the alar base, where the alar base joins with the skin of the superior (upper) lip.

*Subnasal point*: Located on the soft tissue, the point at which the columella merges with the upper lip in the midsagittal plane.

*Supramentale*: The point of greatest concavity in the midline of the lower lip between labrale inferius and soft tissue pogonion

### 6.4.7 Anatomy of the skull

*Frontal bone*: The frontal bone includes a large vertical portion, the *squama frontalis,* corresponding to the region known as the forehead.

*Mandible*: The mandible forms the lower jaw. The mental protuberance is the bony protuberance of the jaw that forms the chin.

*Maxilla*: The maxilla forms the upper jaw and is located above the mandible and below the orbits. The *frontal process* of *the maxilla* projects upwards by the side of the nose, and forms part of its lateral boundary.

*Nasal bones*: The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone*: The occipital bone is situated at the back and lower part of the cranium. It includes an oval aperture, the *foramen agnum,* through which the cranial cavity communicates with the vertebral canal. The curved plate behind the foramen magnum is the *squama occipitalis.*

*Orbit*: The bony cavity in the skull to contain the eyeball.

*Parietal bones*: The parietal bones are the bones that, when joined together, form the roof and sides of the cranium.

*Temporal bones*: The temporal bones are situated on the bases and sides of the skull, and support that part of the face known as the temple.

*Zygomatic bones*: The face includes two zygomatic bones, located in the upper and lateral parts of the face and forming the prominence of the cheek.

### 6.4.8 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx*: The larynx, or voice box houses the vocal folds and connects the inferior part of the pharynx (hypopharynx) with the trachea.

*Lungs*: The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity*: The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx*: The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 6.4.9 Materials

*Silicone or Silicone Elastomer*: A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Coming. Another manufacturer of LSR is Wacker. Unless otherwise specified to the contrary, a preferred form of LSR has a Shore A (or Type A) indentation hardness in the range of about 35 to about 45 as measured using ASTM D2240 (year?required??)

*Polycarbonate*: a typically transparent thermoplastic polymer of Bisphenol-A Carbonate.

### 6.4.10 Aspects of a patient interface

*Anti-asphyxia valve* (*AAV*): The component or sub-assembly of a mask system that, by opening to atmosphere in a failsafe manner, reduces the risk of excessive CO₂ rebreathing by a patient.

*Elbow:* A conduit that directs an axis of flow of air to change direction through an angle. In one form, the angle may be approximately 90 degrees. In another form, the angle may be less than 90 degrees. The conduit may have an approximately circular cross-section. In another form the conduit may have an oval or rectangular cross-section.

*Frame*: Frame will be taken to mean a mask structure that bears the load of tension between two or more points of connection with a headgear. A mask frame may be a non-airtight load bearing structure in the mask. However, some forms of mask frame may also be air-tight.

*Headgear*: Headgear will be taken to mean a form of positioning and stabilizing structure designed for use on a head. Preferably the headgear comprises a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane*: Membrane will be taken to mean a typically thin element that has, preferably, substantially no resistance to bending, but has resistance to being stretched.

*Plenum chamber*: a mask plenum chamber will be taken to a mean portion of a patient interface having walls enclosing a volume of space, the volume having air therein pressurised above atmospheric pressure in use. A shell may form part of the walls of a mask plenum chamber. In one form, a region of the patient's face forms one of the walls of the plenum chamber.

*Seal*: The noun form ("a seal") will be taken to mean a structure or barrier that intentionally resists the flow of air through the interface of two surfaces. The verb form ("to seal") will be taken to mean to resist a flow of air.

*Shell*: A shell will preferably be taken to mean a curved structure having bending, tensile and compressive stiffness, for example, a portion of a mask that forms a curved structural wall of the mask. Preferably, compared to its overall dimensions it is relatively thin. In some forms, a shell may be faceted. Preferably such walls are airtight, although in some forms they may not be airtight.

*Stiffener*: A stiffener will be taken to mean a structural component designed to increase the bending resistance of another component in at least one direction.

*Strut:* A strut will be taken to be a structural component designed to increase the compression resistance of another component in at least one direction.

*Swivel*: (noun) A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. Preferably there is little or no leak flow of air from the swivel in use.

*Tie*: A tie will be taken to be a structural component designed to resist tension.

*Vent*: (noun) the structure that allows a deliberate controlled rate leak of air from an interior of the mask, or conduit to ambient air, to allow washout of exhaled carbon dioxide (CO₂) and supply of oxygen (O₂).

### 6.4.11 Terms used in relation to patient Interface

*Curvature* (*of a surface*): A region of a surface having a saddle shape, which curves up in one direction and curves down in a different direction, will be said to have a negative curvature. A region of a surface having a dome shape, which curves the same way in two principle directions, will be said to have a positive curvature. A flat surface will be taken to have zero curvature.

*Floppy:* A quality of a material, structure or composite that is the combination of features of:
- Readily conforming to finger pressure.
- Unable to retain its shape when caused to support its own weight.
- Not rigid.
- Able to be stretched or bent elastically with little effort.

The quality of being floppy may have an associated direction, hence a particular material, structure or composite may be floppy in a first direction, but stiff or rigid in a second direction, for example a second direction that is orthogonal to the first direction.

*Resilient*: Able to deform substantially elastically, and to release substantially all of the energy upon unloading, within a relatively short period of time such as 1 second.

*Rigid*: Not readily deforming to finger pressure. and/or the tensions or loads typically encountered when setting up and maintaining a patient interface in sealing relationship with an entrance to a patient's airways.

*Semi-rigid*: means being sufficiently rigid to not substantially distort under the effects of mechanical forces typically applied during positive airway pressure therapy.

### 6.5 OTHER REMARKS

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being preferably used to construct a component, obvious alternative materials with similar properties may be used as a substitute. Furthermore, unless specified to the contrary, any and all components herein described are understood to be capable of being manufactured and. as such, may be manufactured together or separately.

It must be noted that as used herein and in the appended claims, the singular forms "a", "an", and "the" include their plural equivalents, unless the context clearly dictates otherwise.

Although the technology herein has been described with reference to particular examples, it is to be understood that these examples are merely illustrative of the principles and applications of the technology. In some instances, the terminology and symbols may imply specific details that are not required to practice the technology.

For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

The scope of the present invention is defined by the following claims.

## Claims

1. A patient interface system to treat a respiratory disorder of a patient, the patient interface system comprising:
a patient interface (3000);
a positioning and stabilising structure (3300) including a back portion (3302) and a pair of upper straps (3312, 3314) extending from the back portion; and
a patient interface frame (3370) for retaining the patient interface against the patient's airways, the patient interface frame including a pair of rigidiser arms (3371, 3381) including a pair of upper attachment points (3375, 3385) each located on one of the pair of rigidiser arms for releasable attachment to a respective upper strap,
wherein when the patient interface system is donned by the patient the rigidiser arms (3371, 3381) are shaped and dimensioned to extend from the patient interface frame (3370) along the patient's cheeks and between the patient's eyes and ears such that each upper strap (3312, 3314) is connectable to a respective upper attachment point (3375, 3385) above the ears of the patient, each upper strap (3312, 3314) being substantially parallel to the patient's Frankfort horizontal when donned by the patient.

2. The patient interface system of claim 1, wherein the patient interface further comprises a plenum chamber (3200) and a seal-forming structure (3100), and optionally, wherein the plenum chamber and the seal-forming structure comprise one piece.

3. The patient interface system of claim 2, wherein the plenum chamber comprises a more rigid material than the seal-forming structure.

4. The patient interface system of any one of claims 2 to 3, wherein the plenum chamber comprises polycarbonate and the seal-forming structure comprises silicone.

5. The patient interface system of any one of claims 2 to 4, wherein the plenum chamber comprises at least one plenum chamber retention feature (3206, 3208).

6. The patient interface system of claim 5, wherein the patient interface frame comprises at least one patient interface frame retention feature (3382, 3384) each configured to releasably attach the patient interface frame to the plenum chamber by a releasable connection with a corresponding one of the at least one plenum chamber retention feature, and
optionally, wherein the plenum chamber comprises four plenum chamber retention features and the patient interface frame comprises four corresponding patient interface frame retention features.

7. The patient interface system of any one of claims 2 to 6, wherein the patient interface frame and the plenum chamber are releasably attachable by a hard-to-hard connection.

8. The patient interface system of any one of claims 1 to 7, wherein the positioning and stabilising structure further comprises a pair of lower straps (3316, 3318) and the patient interface frame further comprises a pair of lower attachment points (3372, 3374) for releasable attachment to a respective lower strap.

9. The patient interface system of claim 8, wherein each of the pair of upper straps and each of the pair of lower straps comprises a loop portion of loop material, and
optionally, wherein a hook portion (3320) comprising hook material is attached at a distal end of each of the pair of upper straps and each of the pair of lower straps.

10. The patient interface system of claim 9, wherein each of the pair of upper straps and each of the pair of lower straps is adapted to be looped around a respective one of the pair of upper attachment points and the pair of lower attachment points such that each respective strap's hook portion is releasably attachable to that strap's loop portion, and/or
wherein each loop portion is wider than each respective hook portion such that when each hook portion is attached to its corresponding loop portion the loop portion shields the patient's skin from the hook portion.

11. The patient interface system of claim 10, further comprising a pair of clips, each of the lower straps being looped through a corresponding one of the clips, and
wherein each of the lower attachment points comprises a receptacle configured to receive a corresponding one of the clips.

12. The patient interface system of any one of claims 1 to 11, wherein the patient interface comprises a full-face mask.

13. The patient interface system of any one of claims 1 to 12, wherein the patient interface does not include a forehead support.

14. The patient interface system of any one of claims 1 to 13, wherein the positioning and stabilising structure comprises a crown strap (3368) to engage with the parietal bone of the patient's skull.

15. The patient interface system of any one of claims 1 to 14, wherein the positioning and stabilising structure comprises a neck piece (3363) to engage with the occipital bone of the patient's skull.

## Patentansprüche

1. Patientenschnittstellensystem zum Behandeln einer Atemwegserkrankung eines Patienten, wobei das Patientenschnittstellensystem aufweist:
eine Patientenschnittstelle (3000);
eine Positionierungs- und Stabilisierungsstruktur (3300) mit einem hinteren Abschnitt (3302) und einem Paar oberer Bänder (3312, 3314), die sich vom hinteren Abschnitt erstrecken; und
einen Patientenschnittstellenrahmen (3370) zum Halten der Patientenschnittstelle an den Atemwegen des Patienten, wobei der Patientenschnittstellenrahmen ein Paar Versteifungsarme (3371, 3381) mit einem Paar oberer Befestigungspunkte (3375, 3385) umfasst, die jeweils an einem der Versteifungsarme des Paars angeordnet sind, um lösbar an einem entsprechenden oberen Band befestigt zu werden,
wobei, wenn das Patientenschnittstellensystem vom Patienten angelegt wird, die Versteifungsarme (3371, 3381) so geformt und dimensioniert sind, dass sie sich vom Patientenschnittstellenrahmen (3370) entlang der Wangen des Patienten und zwischen den Augen und Ohren des Patienten erstrecken, so dass jedes obere Band (3312, 3314) mit einem entsprechenden oberen Befestigungspunkt (3375, 3385) oberhalb der Ohren des Patienten verbunden werden kann, wobei jedes obere Band (3312, 3314) im Wesentlichen parallel zur Frankfurter Horizontalen des Patienten ist, wenn der Patient es angelegt hat.

2. Patientenschnittstellensystem nach Anspruch 1, wobei die Patientenschnittstelle ferner eine Plenumkammer (3200) und eine dichtungsbildende Struktur (3100) aufweist, und wobei die Plenumkammer und die dichtungsbildende Struktur optional ein Stück aufweisen.

3. Patientenschnittstellensystem nach Anspruch 2, wobei die Plenumkammer ein starreres Material als die dichtungsbildende Struktur aufweist.

4. Patientenschnittstellensystem nach einem der Ansprüche 2 bis 3, wobei die Plenumkammer Polycarbonat aufweist und die dichtungsbildende Struktur Silikon aufweist.

5. Patientenschnittstellensystem nach einem der Ansprüche 2 bis 4, wobei die Plenumkammer mindestens ein Plenumkammerhaltemerkmal (3206, 3208) aufweist.

6. Patientenschnittstellensystem nach Anspruch 5, wobei der Patientenschnittstellenrahmen mindestens ein Patientenschnittstellenrahmenhaltemerkmal (3382, 3384) aufweist, das jeweils dafür konfiguriert ist, den Patientenschnittstellenrahmen durch eine lösbare Verbindung mit einem entsprechenden des mindestens einen Plenumkammerhaltemerkmals lösbar an der Plenumkammer zu befestigen, und wobei optional die Plenumkammer vier Plenumkammerhaltemerkmale aufweist und der Patientenschnittstellenrahmen vier entsprechende Patientenschnittstellenrahmenhaltemerkmale aufweist.

7. Patientenschnittstellensystem nach einem der Ansprüche 2 bis 6, wobei der Patientenschnittstellenrahmen und die Plenumkammer durch eine Hart-hart-Verbindung lösbar befestigbar sind.

8. Patientenschnittstellensystem nach einem der Ansprüche 1 bis 7, wobei die Positionierungs- und Stabilisierungsstruktur ferner ein Paar untere Bänder (3316, 3318) aufweist und der Patientenschnittstellenrahmen ferner ein Paar untere Befestigungspunkte (3372, 3374) zum lösbaren Befestigen an einem jeweiligen unteren Band aufweist.

9. Patientenschnittstellensystem nach Anspruch 8, wobei jedes des Paars obere Bänder und jedes des Paars untere Bänder einen Schlaufenabschnitt aus einem Schlaufenmaterial aufweist und wobei optional ein Hakenabschnitt (3320), der ein Hakenmaterial aufweist, an einem distalen Ende jedes des Paars obere Bänder und jedes des Paars untere Bänder befestigt ist.

10. Patientenschnittstellensystem nach Anspruch 9, wobei jedes des Paars obere Bänder und jedes des Paars untere Bänder dazu eingerichtet ist, eine Schleife um einen jeweiligen des Paars oberer Befestigungspunkte und des Paars unterer Befestigungspunkte zu bilden, so dass der Hakenabschnitt eines jeweiligen Bandes lösbar an dem Schlaufenabschnitt dieses Bandes befestigbar ist, und/oder wobei jeder Schlaufenabschnitt breiter als jeder jeweilige Hakenabschnitt ist, so dass, wenn jeder Hakenabschnitt an seinem entsprechenden Schlaufenabschnitt befestigt ist, der Schlaufenabschnitt die Haut des Patienten vor dem Hakenabschnitt schützt.

11. Patientenschnittstellensystem nach Anspruch 10, ferner aufweisend ein Paar Clips, wobei jeder der unteren Bänder eine Schleife durch einen entsprechenden Clip bildet, und
wobei jeder der unteren Befestigungspunkte eine Aufnahme aufweist, die so konfiguriert ist, dass sie einen entsprechenden Clip aufnehmen kann.

12. Patientenschnittstellensystem nach einem der Ansprüche 1 bis 11, wobei die Patientenschnittstelle eine Vollgesichtsmaske aufweist.

13. Patientenschnittstellensystem nach einem der Ansprüche 1 bis 12, wobei die Patientenschnittstelle keine Stirnstütze aufweist.

14. Patientenschnittstellensystem nach einem der Ansprüche 1 bis 13, wobei die Positionierungs- und Stabilisierungsstruktur ein Scheitelband (3368) aufweist, das dazu vorgesehen ist, mit dem Scheitelknochen des Schädels des Patienten in Eingriff zu kommen.

15. Patientenschnittstellensystem nach einem der Ansprüche 1 bis 14, wobei die Positionierungs- und Stabilisierungsstruktur ein Nackenstück (3363) aufweist, das dazu vorgesehen ist, mit dem Hinterhauptknochen des Schädels des Patienten in Eingriff zu kommen.

## Revendications

1. Système d'interface patient pour traiter un trouble respiratoire d'un patient, le système d'interface patient comprenant :
une interface patient (3000) ;
une structure de positionnement et de stabilisation (3300) incluant :
une partie arrière (3302) et
une paire de sangles supérieures (3312, 3314) s'étendant depuis la partie arrière ; et
un cadre d'interface patient (3370) destiné à maintenir l'interface patient contre les voies respiratoires du patient, le cadre d'interface patient comprenant une paire de bras raidisseurs (3371, 3381) comprenant une paire de points de fixation supérieurs (3375, 3385) situés chacun sur l'un des bras raidisseurs de la paire pour être fixées de manière libérable à une sangle supérieure respective,
dans lequel, lorsque le system d'interface patient est enfilé par le patient, les bras raidisseurs (3371, 3381) sont formés et dimensionnés pour s'étendre depuis le cadre d'interface patient (3370) le long des joues du patient et entre les yeux et les oreilles du patient de telle sorte que chaque sangle supérieure (3312, 3314) puisse être reliée à un point de fixation supérieure respectif (3375, 3385) au-dessus des oreilles du patient, chaque sangle supérieure (3312, 3314) étant sensiblement parallèle au plan horizontal de Francfort du patient lorsque le system est enfilé par le patient.

2. Système d'interface patient selon la revendication 1, dans lequel l'interface patient comprend en outre une chambre de distribution (3200) et une structure formant un joint d'étanchéité (3100), et optionnellement dans lequel la chambre de distribution et la structure formant un joint d'étanchéité comprennent une seule pièce.

3. Système d'interface patient selon la revendication 2, dans lequel la chambre de distribution comprend un matériau plus rigide que la structure formant un joint d'étanchéité.

4. Système d'interface patient selon l'une quelconque des revendications 2 à 3, dans lequel la chambre de distribution comprend du polycarbonate et la structure formant un joint d'étanchéité comprend du silicone.

5. Système d'interface patient selon l'une quelconque des revendications 2 à 4, dans lequel la chambre de distribution comprend au moins un élément de retenue de chambre de distribution (3206, 3208).

6. Système d'interface patient selon la revendication 5, dans lequel le cadre d'interface patient comprend au moins un élément de retenue de cadre d'interface patient (3382, 3384), chacun étant configuré pour fixer de manière libérable le cadre d'interface patient à la chambre de distribution par une liaison libérable avec un élément correspondant parmi les au moins un élément de retenue du chambre de distribution, et optionnellement dans lequel la chambre de distribution comprend quatre éléments de retenue de chambre de distribution et le cadre d'interface patient comprend quatre éléments de retenue de cadre d'interface patient correspondants.

7. Système d'interface patient selon l'une quelconque des revendications 2 à 6, dans lequel le cadre d'interface patient et la chambre de distribution peuvent être fixée de manière libérable par une liaison dure-dure.

8. Système d'interface patient selon l'une quelconque des revendications 1 à 7, dans lequel la structure de positionnement et de stabilisation comprend en outre une paire de sangles inférieures (3316, 3318) et le cadre d'interface patient comprend en outre une paire de points de fixation inférieurs (3372, 3374) pour une fixation libérable à une sangle inférieure respective.

9. Système d'interface patient selon la revendication 8, dans lequel chacune de la paire de sangles supérieures et chacune de la paire de sangles inférieures comprend une partie boucle de matériau de boucle, et optionnellement une partie crochet (3320) comprenant un matériau de crochet est fixée au niveau d'une extrémité distale de chacune de la paire de sangles supérieures et de chacune de la paire de sangles inférieures.

10. Système d'interface patient selon la revendication 9, dans lequel chacune de la paire de sangles supérieures et chacune de la paire de sangles inférieures est adaptée pour former des boucles autour de l'un respectif de la paire de points de fixation supérieurs et de la paire de points de fixation inférieurs de telle sorte que la partie crochet de chaque sangle respective peut être fixée de manière libérable à la partie boucle de cette sangle, et/ou dans lequel chaque partie boucle est plus large que chaque partie crochet respective de telle sorte que, lorsque chaque partie crochet est fixée à sa partie boucle correspondante, la partie boucle protège la peau du patient de la partie crochet.

11. Système d'interface patient selon la revendication 10, comprenant en outre une paire de clips, chacune des sangles inférieures étant bouclée à travers un clip correspondant, et
dans lequel chacun des points de fixation inférieurs comprend un réceptacle configuré pour recevoir un clip correspondant.

12. Système d'interface patient selon l'une quelconque des revendications 1 à 11, dans lequel l'interface patient comprend un masque intégral.

13. Système d'interface patient selon l'une quelconque des revendications 1 à 12, dans lequel l'interface patient ne comprend pas de support frontal.

14. Système d'interface patient selon l'une quelconque des revendications 1 à 13, dans lequel la structure de positionnement et de stabilisation comprend une sangle couronne (3368) pour entrer en prise avec l'os pariétal du crâne du patient.

15. Système d'interface patient selon l'une quelconque des revendications 1 à 14, dans lequel la structure de positionnement et de stabilisation comprend une pièce de cou (3363) pour entrer en prise avec l'os occipital du crâne du patient.
